# EUROPEAN PATENT SPECIFICATION

(11) **EP 2 087 138 B1**
(45) Date of publication and mention of the grant of the patent: **17.07.2013**
(21) Application number: 07847212.3
(22) Date of filing: 19.11.2007
(51) Int. Cl.: C12Q 1/68

(54) **MICROARRAY FOR THE DETECTION OF AN ANGIOSTATIC TUMOR STAGE OF COLORECTAL CARCINOMA**
MIKROARRAY ZUM NACHWEIS EINES ANGIOSTATISCHEN KOLOREKTALKARZINOM-TUMORSTADIUMS
MICRORÉSEAU POUR LA DÉTECTION D'UN STADE DE TUMEUR ANGIOSTATIQUE DU CARCINOME COLORECTAL

(30) Priority: 29.11.2006 US 861624 P
(43) Date of publication of application: 12.08.2009
(73) Proprietor: Friedrich-Alexander-Universität Erlangen-Nürnberg, 91054 Erlangen (DE)
(72) Inventor: CRONER, Roland S., 91341 Roettenbach (DE); STUERZL, Michael, 91054 Erlangen (DE); NASCHBERGER, Elisabeth, 91055 Erlangen (DE)
(74) Representative: Isarpatent
(86) International application number: PCT/EP2007/062522
(87) International publication number: WO 2008/065020

(56) References cited:
- CRONER R.S. ET AL.: "Microarray versus conventional prediction of lymph node metastasis in colorectal carcinoma" CANCER, AMERICAN CANCER SOCIETY, PHILADELPHIA, PA, US, vol. 104, no. 2, 10 June 2005 (2005-06-10), pages 395-404, XP009088697 ISSN: 0008-543X
- "GeneChip Human Genome U133 set" ANNOUNCEMENT AFFYMETRIX, September 2001 (2001-09), XP002324895
- ROMAGNANI P. ET AL.: "CXC chemokines: the regulatory link between inflammation and angiogenesis" TRENDS IN IMMUNOLOGY, ELSEVIER, RAHWAY, NJ, US, vol. 25, no. 4, April 2004 (2004-04), pages 201-209, XP004496713 ISSN: 1471-4906 cited in the application
- LUBESEDER-MARTELLATO C. ET AL.: "GUANYLATE-BINDING PROTEIN-1 EXPRESSION IS SELECTIVELY INDUCED BY INFLAMMATORY CYTOKINES AND IS AN ACTIVATION MARKER OF ENDOTHELIAL CELLS DURING INFLAMMATORY DISEASES" AMERICAN JOURNAL OF PATHOLOGY, PHILADELPHIA, PA, US, vol. 161, no. 5, November 2002 (2002-11), pages 1749-1759, XP009029105 ISSN: 0002-9440 cited in the application
- PRAKASH B. ET AL.: "Structure of human guanylate-binding protein 1 representing a unique class of GTP-binding proteins" NATURE (LONDON), vol. 403, no. 6769, 3 February 2000 (2000-02-03), pages 567-571, XP002474122 ISSN: 0028-0836 cited in the application
- GREENBAUM D. ET AL.: "COMPARING PROTEIN ABUNDANCE AND MRNA EXPRESSION LEVELS ON A GENOMIC SCALE" GENOME BIOLOGY (ONLINE), GB, vol. 40, no. 9, 2003, pages 11701-11708, XP008036618 ISSN: 1465-6914
- GREENBAUM D. ET AL.: "Interrelating different types of genomic data, from proteome to secretome: 'Oming in on function" GENOME RESEARCH, COLD SPRING HARBOR LABORATORY PRESS, WOODBURY, NY, US, vol. 11, no. 9, September 2001 (2001-09), pages 1463-1468, XP009088703 ISSN: 1088-9051
- OERNTOFT T.F. ET AL.: "GENOME-WIDE STUDY OF GENE COPY NUMBERS, TRANSCRIPTS, AND PROTEIN LEVELS IN PAIRS OF NON-INVASIVE AND INVASIVE HUMAN TRANSITIONAL CELL CARCINOMAS" MOLECULAR AND CELLULAR PROTEOMICS, AMERICAN SOCIETY FOR BIOCHEMISTRY AND MOLECULAR BIOLOGY, US, vol. 1, no. 1, 2002, pages 37-45, XP008015037 ISSN: 1535-9476
- CELIS A .J.E. ET AL.: "Gene expression profiling: monitoring transcription and translation products using DNA microarrays and proteomics" FEBS LETTERS, ELSEVIER, AMSTERDAM, NL, vol. 480, no. 1, 25 August 2000 (2000-08-25), pages 2-16, XP004337487 ISSN: 0014-5793

## Description

The present invention is directed to a microarray for the detection of an angiostatic tumor stage/tumor area of colorectal carcinoma in a patient, wherein the microarray comprises gene probes capable of specifically hybridizing to predefined nucleic acids. The invention is further directed to an inhibitor or modulator of one or more of these nucleic acids, as well as to a pharmaceutical composition, comprising those inhibitors or modulators. In a further aspect, the present invention is directed to an *ex vivo* method for the diagnosis of an angiostatic tumor stage/tumor area in a patient suffering from a colorectal carcinoma. In a further aspect the invention is directed to predict the response of patients with colorectal carcinoma but also other diseases to therapy.

### Background of the invention

Colorectal Cancer is the third most frequently occurring cancer in both sexes worldwide. It ranks second in developed countries (Hawk and Levin, 2005). The cumulative life time risk of developing colorectal cancer is about 6 % (Smith et al., 2002). Despite the advances in the treatment of this disease the 5-year survival is only 62 % (Smith et al., 2002).

Three pathways have been described as the basis for malignant transformation within the colon. These are the chromosomal instability pathway, the microsatellite instability pathway (Vogelstein et al., 1988) and the methylation pathway (Jass, 2002).

Malignant transformation of the colorectal epithelium typically occurs as a multistep process that requires cumulative damage to different genes within several cellular generations. Initially cryptal hyperplasia, a proliferation of normal-appearing cells, commonly results from genetic or epigenetic changes in pathways regulating cell cycle progression or apoptosis such as APC or Bcl-2 (Baylin and Herman, 2000). The transition from hyperproliferation to dysplasia is characterized by abnormal nuclear and/or cellular shapes in crypts with larger cells, often characterized by mutations in k-ras (Takayama et al., 2001). Progression from these aberrant crypt foci to adenoma, and subsequently to carcinoma, is typically associated with additional aberrations involving SMAD-2/4, DCC, and p53 (Ilyas et al., 1999). In addition to the genetic changes in the tumor cells two important stroma reactions are associated with colorectal cancer pathogenesis: angiogenesis and inflammation.

### Angiogenesis in Colorectal Carcinoma:

Tumor growth beyond the critical two to three millimeter diameter and metastasis require angiogenesis. The important role of angiogenesis in colorectal cancer progression has been convincingly documented. It has been shown that microvessel density increases around primary tumors compared with normal mucosa or adenomas (Bossi et al., 1995), and is a strong independent predictor of poor outcome (Takebayashi et al., 1996). High microvessel density is associated with a greater than 3-fold risk of death from colorectal cancer (Choi et al., 1998). In addition, vascular endothelial growth factor (VEGF) expression is significantly increased in patients with all stages of colorectal carcinoma as compared to controls (Kumar et al., 1998). Intratumor expression of VEGF was found to be associated with a nearly 2-fold increase of death risk from colorectal cancer (Ishigami et al., 1998) and correlated with increasing tumor stage, decreased overall survival, and decreased disease-free survival (Kahlenberg et al., 2003; Kang et al., 1997). Recently, all of these observations were convincingly supported in a clinical study. In this study an anti-VEGF antibody (Bevacizumab, Avastin) was added to flourouracil-based combination chemotherapy. This approach resulted in statistically significant and clinically meaningful improvement in survival among patients with metastatic colorectal cancer (Hurwitz et al., 2004). This was the first report on successful tumor therapy with antiangiogenic treatment strategies, which clearly documented the importance of angiogenesis in colorectal cancer pathogenesis.

### Endothelial cell and inflammatory cell interaction:

As yet, the effect of inflammation on angiogenesis in colorectal carcinoma has not been investigated in detail. Blood vessels can be detected in inflammatory areas of colorectal carcinomas. In addition, angiogenesis is a characteristic feature of inflammatory tissues. Both observations apparently suggest that inflammation may positively contribute to angiogenesis in colorectal carcinoma. However, it is well known that inflammatory cytokines such as interleukin (IL)-1beta, tumor necrosis factor (TNF)-alpha and interferon (IFN)-gamma are potent inhibitors of endothelial cell proliferation and invasion *in vitro* (Cozzolino et al., 1990; Frater-Schroder et al., 1987; Friesel et al., 1987; Guenzi et al., 2001; Guenzi et al., 2003; Schweigerer et al., 1987). In addition, inflammatory cytokines have been shown to inhibit angiogenesis in different animal models *in vivo* (Cozzolino et al., 1990; Fathallah-Shaykh et al., 2000; Norioka et al., 1994; Yilmaz et al., 1998). In contrast, in some other animal models an induction of angiogenesis has been observed in the presence of inflammatory cytokines (Frater-Schroder et al., 1987; Gerol et al., 1998; Mahadevan et al., 1989; Montrucchio et al., 1994; Torisu et al., 2000) and it has been reported that according to their concentrations inflammatory cytokines may act either as pro- or anti-angiogenic molecules in the same model system (Fajardo et al., 1992).

The antiangiogenic effect of inflammatory cytokines may be caused by their direct inhibitory effects on endothelial cell proliferation and invasion (Guenzi et al., 2001; Guenzi et al., 2003; Naschberger et al., 2005). The angiogenic effects of inflammatory cytokines have been attributed to indirect mechanisms, via the recruitment of monocytes into tissues that in turn may release angiogenic factors (Fajardo et al., 1992; Frater-Schroder et al., 1987; Joseph and Isaacs, 1998; Montrucchio et al., 1994) or to the induction of basic fibroblast growth factor (bFGF) or VEGF expression in resident cells (Samaniego et al., 1997; Torisu et al., 2000). Altogether, these results indicate that angiogenesis in colorectal carcionoma may critically depend on the specific micromilieu generated by the interplay of tumor cells, inflammatory cells and endothelial cells. This may significantly vary in different tumor stages but also in different areas of the same tumor. Thus, angiogenesis may be activated in certain tumor areas/stages and inhibited in others.

The relationship of inflammation and cancer has been a matter of debate up to now. Chronic inflammatory diseases such as ulcerative colitis and Crohn's disease predispose patients for colorectal carcinoma with an up to 10-fold increased risk (reviewed in Itzkowitz and Yio, 2004; Clevers, 2004; Farrell and Peppercorn, 2002). It has been demonstrated that chronic inflammation not only triggers the progression of cancer but also the initiation. For example, chronic inflammation is believed to be responsible for the neoplastic transformation of intestinal epithelium (reviewed in Itzkowitz and Yio, 2004). In contrast, acute inflammation of the Th1-type is considered as a host response which antagonizes tumor progression. Efforts have been undertaken to induce acute inflammation in tumor patients by e.g. systemic IL-2 immunotherapy in renal cell carcinoma where but the responses were low (Negrier et al., 1998). The relationship of inflammation, tumor initiation/progression and angiogenesis in the sporadic CRC remains largely unclear.

Recently, a concept determined as "immunoangiostasis" has been introduced by Strieter and colleagues. It was described that under certain pathological conditions in the tissue a micromilieu is established that corresponds to an IFN-γ-dependent (Th-1-like) immune reaction which finally leads to an intrinsic angiostatic reaction. This angiostatic activity has been largely attributed to the induction of the anti-angiogenic chemokines CXCL9 (monokine induced by IFN-γ [MIG]), CXCL10 (IFN-γ inducible protein-10 [IP-10]) and CXCL11 (IFN-inducible T-cell a chemoattractant [I-TAC]) by IFN-γ These chemokines belong to the CXC chemokine subfamily that all lack a so called "ELR" amino acid motif (Glu-Leu-Arg) (Strieter et al., 2005b). Currently, the anti-angiogenic chemokines consist of five members that are CXCL4 (platelet factor-4 [PP-4]) (Spinetti et al., 2001), CXCL9, CXCL10, CXCL11 and CXCL13 (B-cell chemoattractant-1 [BCA-1]) (Romagnani et al., 2004). All angiostatic chemokines except from CXCL4 are induced by IFN-gamma (Romagnani et al., 2001). CXCL4, CXCL9, CXCL10 and CXCL11 bind to the same receptor, namely CXCR3 that is expressed by CD4 and CD8 lymphocytes, B cells, NK cells and endothelial cells. The CXCR3 receptor exists in two alternatively spliced variants CXCR3-A and CXCR3-B and the latter is responsible for the anti-angiogenic action of the chemokines (Lasagni et al., 2003).

One of the most abundant proteins induced by IFN-γ is the guanylate binding protein-1 (GBP-1) that belongs to the family of large GTPases (Prakash et al., 2000; Cheng et al., 1983; Naschberger et al., 2005).

The inventors demonstrated that GBP-1 is not only induced by IFN-γ, rather by a group of inflammatory cytokines (IFN-α/γ, interleukin [IL]-1α/β and tumor necrosis factor [TNF]-α) (Lubeseder-Martellato et al., 2002; Naschberger et al., 2004). GBP-1 expression was preferentially associated with endothelial cells (EC) *in vitro* and *in vivo* (Lubeseder-Martellato et al., 2002) and GBP-1 was shown to regulate and mediate the inhibition of proliferation induced by inflammatory cytokines (IC) in endothelial cells as well as their invasive capacity (Guenzi et al., 2001; Guenzi et al., 2003). The protein was established as a histological marker of normal endothelial cells that are activated by IC and display an anti-angiogenic phenotype.

Thus, inflammation and angiogenesis are important stroma reactions of colorectal carcinoma (CRC). Inflammation can exert pro- or antiangiogenic activity. These effects of inflammation may vary in different patients. Pre-therapeutic differentiation of angiogenic and angiostatic inflammation therefore may clearly improve the efficacy of antiangiogenic but also of other forms of therapy of CRC. In addition, this approach may also be adequate to predict therapy response in other diseases.

### Summary of the invention

Therefore, it is an object of the invention to provide a means and method for the detection, prediction and/or diagnosis of an angiostatic tumor stage/tumor area of colorectal carcinoma in a patient. It is a further object ot the present invention to provide molecular markers to predict responses to therapy of patients with colorectal carcinoma and also other diseases (e.g. breast carcinoma, lung canarcinoma also). It is a further object of the present invention to provide substances, which are suitable for the treatment of colorectal carcinoma.

These objects are achieved by the subject-matter of the independent claims. Preferred embodiments are set forth in the dependent claims.

The inventors investigated whether guanylate binding protein-1 (GBP-1) may be a marker of angiostatic inflammation in CRC, because it characterizes endothelial cells exposed to inflammatory cytokines and mediates the direct antiangiogenic effects of these factors.

It was found that GBP-1 is strongly expressed in endothelial cells and monocytes in the desmoplactic stroma of some CRC. Transcriptome analysis of GBP-1-positive and -negative CRC (n=24) demonstrated that GBP-1 is highly significant (p < 0.001) associated with an interferon-γ (IFN-γ)-dominated micromilieu and high expression of antiangiogenic chemokines (CXCL9, CXCL10, CXCL11). Corresponding conditions have been referred to as immunoangiostasis (IAS) recently. The association of GBP-1 and angiostasis was confirmed by the detection of an inverse relation of GBP-1 expression and endothelial cell proliferation in the tumor vessels. Moreover, this association was affirmed in an independent disease, namely caseating tuberculosis. This avascular disease is the prototype of highly active IAS and exhibited an extremely robust expression of GBP-1. Most importantly, an immunohistochemical analysis of 388 colonic carcinoma tissues showed that GBP-1 was associated with a highly significant (p<0.001) increased (16.2%) cancer-related 5-year survival of the patients. Moreover, the relative risk of cancer-related death was lowered by 50% in GBP-1-positive colonic carcinoma.

It is shown herein that GBP-1 is a novel marker, among others, and active component of IAS in CRC and it is demonstrated that GBP-1-associated IAS is beneficial for the survival of CRC patients. GBP-1 expression along with the coexpression of several other markers may be a valuable prognostic marker to identify tumors with high intrinsic antiangiogenic activity and GBP-1-positive CRC will differentially respond to antiangiogenic therapy but also to all other forms of therapy as compared to GBP-1-negative CRC. The induction of GBP-1-associated IAS may be a promising approach for the clinical treatment of CRC.

At present an angiostatic stage is not considered to exist in CRC. The inventors have demonstrated that such a stage exists, concommitantly with the availability of means and methods, which allow to detect this stage.

The availability of a method to detect patients with "angiostatic CRC" has three major advantages: (1) It allows at an early stage to apply appropriate treatment strategies to these patients. (2) The specific selection of patients will improve the clinical efficacy of antiangiogenic therapy but likely also to other forms of therapy. (3) Improved selection criteria for therapy responsive patients will significantly reduce the costs for the health system.

Specific forms of therapy which are referred to above include the following but also additional drugs which are used for treatment of colorectal carcinoma but also additional diseases:
(1) Direct and indirect inhibitors of angiogenesis, immunomodulatory molecules and other drugs (clinically approved): monoclonal antibodies (e.g. bevacizumab, cetuximab, ranibizumab, panitumumab), tyrosine kinase inhibitors (e.g. erlotinib, sunitinib/SU11248, sorafenib, temsirolimus), aptamers (e.g. pegaptanib), endogenous angiogenesis inhibitors (e.g. endostatin), thalidomide, paclitaxel, celecoxib, bortezomib, trastuzumab, lenalidomid.
(2) Direct and indirect inhibitors of angiogenesis, immunomodulatory molecules and other drugs (clinically non-approved, in clinical trial): e.g. PTK787, SU5416, ABT-510, CNGRC peptide TNF-alpha conjugate, cyclophosphamide, combretastatin A4 phosphate, dimethylxanthenone acetic acid, docetaxel, LY317615, soy isoflavone, ADH-1, AG-013736, AMG-706, AZD2171, BMS-582664, CHIR-265, pazopanib, PI-88, everolimus, suramin, XL184, ZD6474, ATN-161, cilenigtide.

Altogether, the invention will contribute to predict therapy responses to a variety of different drugs in different diseases. In addition,_the invention will contribute an important tool to the development of improved treatment strategies for cancer, which are considering the specific cellular activation phenotype predominating in individual patients to gain optimal therapeutic success.

### Detailed description of the invention

According to a first aspect, the present invention provides a microarray for the detection of an angiostatic tumor stage/tumor area of colorectal carcinoma in a patient, wherein the microarray comprises gene probes capable of specifically hybridizing to the nucleic acids according to Seq. No. 1-108 or derivatives thereof, wherein the array comprises gene probes hybridizing to a subset of at least 4 of the above nucleic acid sequences, and further, wherein the array comprises gene probes specifically hybridizing to the nucleic acid sequences of Seq. No. 1, 4, 8 and 41.

The term "microarray" as used herein is meant to comprise DNA microarrays as well as protein microarrays.

A DNA microarray in the meaning of the present invention (also commonly known as gene or genome chip, DNA chip, or gene array) is a collection of microscopic DNA spots attached to a solid surface, such as glass, plastic or silicon chip forming an array for the purpose of expression profiling, monitoring expression levels for several genes simultaneously.

The affixed DNA segments are known and termed herein as probes, and many of them can be used in a single DNA microarray. The term gene probe generally means a specific sequence of single-stranded DNA or RNA. The term "probe" generally is here defined as a nucleic acid which can bind to a target nucleic acid via one or more kind of chemical binding, usually via complementary base pairing which usually utilizes hydrogen bonds. A probe thus is designed to bind to, and therefore single out, a particular segment of DNA to which it is complementary. Therefore, it is sufficient for the purposes of the present invention that the gene probe only hybridizes to a small part of the nucleic acid sequences indicated herein.

For performing an analysis, the following approach might be chosen:
At first, RNA is extracted from a patient sample, than the RNA is transcribed into cDNA or cRNA following purification and/or amplification steps. The cDNA or cRNA obtained may be provided with labels, if required. These nucleic acids in the next step are hybridized with the microarray as defined herein, whereby labelled cDNA or cRNA pieces are binding to its complementary counterpart on the array. Following washing away unbound cDNA or cRNA pieces, the signal of the labels in each position of the microarray may be recorded by a suitable device.

As mentioned above and as it can be derived from Table 4, GBP-1 (Seq. No. 41) is a powerful biomarker of an angiostatic immune reaction in colorectal cancer (CRC) and might already serve alone as a valuable tool for detecting an angiostatic tumor stage in a patient suffering from CRC. However, it also turned out that an even more valuable tool can be established, if the expression of at least three additional markers is evaluated, being the genes corresponding to Seq. No. 1, 4, and 8 (CXCL11, CXCL9 and CXCL 10). Interestingly, these three chemokines CXCL9, CXCL10, CXCL11 were among the 15 highest upregulated genes in GBP-1-positive tumors and were also found to be clearly higher expressed in GBP-1-positive as compared to -negative tumors. Thus, they can serve to enhance the sensitivity of detecting an angiostatic stage in an individual patient.

Therefore, it is an essential element of the invention that the microarray is at least comprising gene probes which are capable of hybridizing to the nucleic acid sequences of Seq. No. 1, 4, 8 and 41.

Although it is sufficient that the array contains these probes in order to achieve the object of the present invention, i.e. to detect, whether an angiostatic stage is present in an individual CRC patient or not (in order to subsequently chose the appropriate therapeutical steps), additional gene probes may be included which are capable of hybridizing to further nucleic acids selected from the group of Seq. No. 1-108.

Among these, further subgroups of genes preferably may be selected, specifically those, which are expressed in increased levels in GBP-1-positive CRC and have been shown to play an important role in the regulation of the cellular response to IFN: 1, 4, 8, 14, 25, 26, 41, 54 59, 65, 76, 81, 105, 106, 107, 108 and those whose expression is more than 10fold increased in GBP-1 positive CRC: 1 - 17. Further subgroups may be identified as Seq. No. 26, 54, 59, 65, 81, 105, 106, 107 and/or 108. It is noted that it is also preferred to additionally use these nucleic acids alone or in combination which each other, for example, and more preferred, subgroups Seq. No. 26, 54, 59, 65, 81 and/or 105, 106, 107, 108.

In a further embodiment, the microarray may additionally contain gene probes capable of specifically hybridizing to at least one of the nucleic acids according to Seq. No. 109-157, being 49 gene probes of genes with increased expression in hGBP-1-negative CRC (see the genes indicated in Table 5. Seq. No.'s correspond to the order of the sequences indicated in the table starting from Seq. No. 109). These additional nucleic acid sequences and the respective gene probes hybridizing to them may be used as "negative" control in order to further enhance the predictive value of the microarray.

Because it has been shown that vascular endothelial cell growth factor (VEGF) and basic fibroblast growth factor (bFGF) are major regulators of angiogenesis, the microarray may preferably also contain probes also to these genes. Both genes were not found to be differentially expressed in GBP-1-positive and -negative CRC, because they are generally expressed in increased levels in all CRC as compared to healthy tissues. However, due to their specific activity which antagonizes the effects of GBP-1-associated immunoangiostasis, probes for VEGF (incuding VEGF-A, VEGF-B, VEGF-C, VEGF-D) and bFGF and all splice variants of the respective genes will be used as a standard to determine basic angiogenic activation. To these goal the probes for VEGF and bFGF will be applied in combination with all gene groups mentioned above: namely 1-108 or 109-157, or 1, 4, 8, 14, 25, 26, 41, 59, 6S, 76, 81, 105, 106,107,108 or 1 -17.

The microarray of the present invention additionally may contain appropriate control gene probes, e.g. actin or GAPDH. Those can be included as contol gene probes to determine relative signal intensities.

In a preferred embodiment, the gene probes used in the microarray of the invention are oligonucleotides, cDNA, RNA or PNA molecules.

As mentioned above, the nucleic acids as defined above preferably are labelled in order to allow a better detection of their binding to the corresponding gene probe on the array. Preferably, such a label is selected from the group consisting of a radioactive, fluorescence, biotin, digoxigenin, peroxidase labelling or a labelling detectable by alkaline phosphatase.

In a further embodiment, the gene probes of the array may be bound to a solid phase matrix, e.g. a nylon membrane, glass or plastics.

In a second aspect, the present invention is directed to a protein microarray, capable of detecting at least a subset of four amino acid sequences of a group of amino acid sequences corresponding to the nucleic acid sequences of Seq. No. 1-108, wherein the array is capable of at least detecting the amino acids corresponding to the nucleic acid sequences of Seq. No. 1, 4, 8 and 41.

Or in other words, the protein microarray is capable of detecting all amino acids corresponding to nucleic acid sequences and subgroups as defined hereinabove.

In the protein microarray of the present invention, the array preferably is an antibody microarray or a Western-blot microarray.

An antibody microarray is a specific form of a protein microarray, i.e. a collection of capture antibodies are spotted and fixed on a solid surface, such as glass, plastic and a silicon chip for the purpose of detecting antigens.

The term "antibody", is used herein for intact antibodies as well as antibody fragments, which have a certain ability to selectively bind to an epitope. Such fragments include, without limitations, Fab, F(ab')₂, ScFv and Fv antibody fragment. The term "epitop" means any antigen determinant of an antigen, to which the paratop of an antibody can bind. Epitop determinants usually consist of chemically active surface groups of molecules (e.g. amino acid or sugar residues) and usually display a three-dimensional structure as well as specific physical properties.

The antibodies according to the invention can be produced according to any known procedure. For example the pure complete protein according to the invention or a part of it can be produced and used as immunogen, to immunize an animal and to produce specific antibodies.

The production of polyclonal antibodies is commonly known. Detailed protocols can be found for example in Green et al, Production of Polyclonal Antisera, in Immunochemical Protocols (Manson, editor), pages 1 - 5 (Humana Press 1992) und Coligan et al, Production of Polyclonal Antisera in Rabbits, Rats, Mice and Hamsters, in Current Protocols In Immunology, section 2.4.1 (1992). In addition, the expert is familiar with several techniques regarding the purification and concentration of polyclonal antibodies, as well as of monoclonal antibodies (Coligan et al., Unit 9, Current Protocols in Immunology, Wiley Interscience, 1994).

The production of monoclonal antibodies is as well commonly known. Examples include the hybridoma method (Kohler and Milstein, 1975, Nature, 256:495-497, Coligan et al., section 2.5.1 - 2.6.7; and Harlow et al., Antibodies: A Laboratory Manual, page 726 (Cold Spring Harbor Pub. 1988).), the trioma technique, the human B-cell hybridoma technique (Kozbor et al., 1983, Immunology Today 4:72), and the EBV-hybridoma technique to produce human monoclonal antibodies (Cole et al., 1985, in Monoclonal Antibodies and Cancer Therapy, Alan R. Liss, Inc., pp. 77-96).

In brief, monoclonal antibodies can be attained by injecting a mixture which contains a protein/peptide into mice/rats. The antibody production in the mice/rats is checked via a serum probe. In the case of a sufficient antibody titer, the mouse/rat is sacrificed and the spleen is removed to isolate B-cells. The B cells are fused with myeloma cells resulting in hybridomas. The hybridomas are cloned and the clones are analyzed. Positive clones which contain a monoclonal antibody against the protein are selected and the antibodies are isolated from the hybridoma cultures. There are many well established techniques to isolate and purify monoclonal antibodies. Such techniques include affinity chromatography with protein A sepharose, size-exclusion chromatography and ion exchange chromatography. Also see for example, Coligan et al., section 2.7.1 - 2.7.12 and section "Immunglobulin G (IgG)", in Methods In Molecular Biology, volume 10, pages 79-104 (Humana Press 1992).

In a third aspect, the present invention provides an inhibitor or modulator of one or more of the nucleic acids of Seq. No. 1-108, or of the amino acids expressed therefrom. Such substances may be used for the treatment of colorectal carcinoma.

The inhibitor or modulator is preferably selected from the group consisting of an antisense nucleic acid, a ribozyme, double stranded RNA, siRNA, microRNA an antibody, a receptor, a mutated transdominant negative variant of the protein, a peptide and a peptidomimetic.

In a fourth aspect, the invention provides a pharmaceutical composition, which comprises an inhibitor/modulator as defined above and a pharmaceutically acceptable carrier.

The active compounds of the present invention are preferably used in such a pharmaceutical composition, in doses mixed with an acceptable carrier or carrier material, that the disease can be treated or at least alleviated. Such a composition can (in addition to the active component and the carrier) include filling material, salts, buffer, stabilizers, solubilizers and other materials, which are known state of the art.

The term "pharmaceutically acceptable" is defined as non-toxic material, which does not interfere with effectiveness of the biological activity of the active compound. The choice of the carrier is dependent on the application.

The pharmaceutical composition can contain additional components which enhance the activity of the active component or which supplement the treatment. Such additional components and/or factors can be part of the pharmaceutical composition to achieve a synergistic effects or to minimize adverse or unwanted effects.

Techniques for the formulation or preparation and application/medication of compounds of the present invention are published in "Remington's Pharmaceutical Sciences", Mack Publishing Co., Easton, PA, latest edition. A therapeutically effective dose relates to the amount of a compound which is sufficient to improve the symptoms, for example a treatment, healing, prevention or improvement of such conditions. An appropriate application can include for example oral, dermal, rectal, transmucosal or intestinal application and parenteral application, including intramuscular, subcutaneous, intramedular injections as well as intrathecal, direct intraventricular, intravenous, intraperitoneal or intranasal injections. The intravenous injection is the preferred treatment of a patient.

A typical composition for an intravenous infusion can be produced such that it contains 250 ml sterile Ringer solution and for example 10 mg protein compound. See also Remington's Pharmaceutical Science (15. edition, Mack Publishing Company, Easton, Ps., 1980).

The active component or mixture of it in the present case can be used for prophylactic and/or therapeutic treatments.

A fifth aspect of the present invention is directed to an *ex vivo* method for the diagnosis of an angiostatic tumor stage/tumor area in a CRC patient comprising the steps of:
a) providing a sample of the patient;
b) extracting RNA from the sample;
c) optional ly transcribing RNA to cDNA or cRNA;
d) detecting, whether at least four nucleic acid sequences selected from the group consisting of Seq. No. 1-108 are present in the sample, and whether the sample contains at least the nucleic acid sequences of Seq. No. 1, 4, 8 and 41;
e) wherein the presence of said nucleic acids is indicative for the presence of an angiostatic tumor stage/tumor area of CRC in said patient.

The sample used in this method preferably is a CRC tissue sample or a cell lysate or a body fluid sample.

The detection preferably is performed by PCR, more preferably by RT-PCR, most preferably multiplex RT-PCR. The PCR method has the advantage that very small amounts of DNA are detectable. Dependent on the to be analyzed material and the equipment used the temperature conditions and number of cycles of the PCR have to be adjusted. The optimal conditions can be experimentally determined according to standard procedures.

Multiplex-PCR conditions for the simultaneous detection of GBP-1, CXCL9, CXCL10 and CXCL11 might be set as follows:
Reaction mixture:
   cDNA 1 µl (corresponding to 50 ng total-RNA)
   dNTP 200 µM
   GBP-1, CXCL10 and CXCL11 primer each 0.4 µM, CXCL9 primer 0.8 µM
   10x FastStart High Fidelity Reaction Buffer (Fa. Roche) 5 µl
   FastStart High Fidelity Enzyme (Fa. Roche) 0,5 µl
   Ad 50 µl Millipore-H₂O
Program:
   95°C 2 min 1x
   95°C 30 sec 35x
   55°C 30 sec
   72°C 30 sec
   72°C 4 min 1x
   4°C unlimited

1/3 of the PCR-product are applied to a agarose gel.

The during the PCR amplification accrued, characteristic, specific DNA fragments can be detected for example by gel electrophoretic or fluorimetric methods with the DNA labeled accordingly. Alternatively, other appropriate, known to the expert, detection systems can be applied.

The DNA or RNA, especially mRNA, of the to be analyzed probe can be an extract or a complex mixture, in which the DNA or RNA to be analyzed are only a very small fraction of the total biological probe. This probe can be analyzed by PCR, e.g. RT-PCR. The biological probe can be serum, blood or cells, either isolated or for example as mixture in a tissue.

The detection is - as already outlined above - preferably performed by means of complementary gene probes. Those gene probes preferably are cDNA or oligonucleotide probes. Furthermore, these gene probes preferably are capable of hybridizing to at least a portion of the nucleic acid sequences of Seq. No. 1-108, or to RNA sequences or derivatives derived therefrom.

According to the invention, the hybridization to the nucleic acids according to the invention is done at moderate stringent conditions.

Stringent hybridization and wash conditions are in general the reaction conditions for the formation of duplexes between oligonucleotides and the desired target molecules (perfect hybrids) or that only the desired target can be detected. Stringent washing conditions mean 0.2 x SSC (0.03 M NaCl, 0.003 M sodium citrate, pH 7)/0.1 % SDS at 65°C. For shorter fragments, e.g. oligonucleotides up to 30 nucleotides, the hybridization temperature is below 65°C, for example at 50°C, preferably above 55°C, but below 65°C. Stringent hybridization temperatures are dependent on the size or length, respectively of the nucleic acid and their nucleic acid composition and will be experimentally determined by the skilled artisan. Moderate stringent hybridization temperatures are for example 42°C und washing conditions with 0.2 x SSC/0.1% SDS at 42°C.

The expert can according to the state of the art adapt the chosen procedure, to reach actually moderate stringent conditions and to enable a specific detection method. Appropriate stringent conditions can be determined for example on the basis of reference hybridization. An appropriate nucleic acid or oligonucleotide concentration needs to be used. The hybridization has to occur at an appropriate temperature (the higher the temperature the lower the binding).

In a preferred embodiment, the microarray as defined above is used for the detection.

A sixth aspect of the present invention provides an *ex vivo* method for the diagnosis of an angiostatic tumor stage/tumor area in a CRC patient comprising the steps of:
a) providing a sample from the patient;
b) detecting, whether at least four amino acid sequences corresponding to the nucleic acid sequences selected from the group of Seq. No. 1-108 are present in the sample, and whether the sample contains at least the amino acids corresponding to the nucleic acid sequences of Seq. No. 1, 4, 8 and 41;
c) wherein the presence of said proteins is indicative for the presence of an angiostatic tumor stage/tumor area of CRC in said patient.

In a preferred embodiment, the detection is performed by contacting the sample with antibodies, which specifically recognize an amino acid expressed from a nucleic acid sequence of one of Seq. No. 1-108.

Preferably, the sample is a CRC tissue sample, a cell lysate or a body fluid. The amino acid sequences are preferably detected by means of multiplex Western blot or ELISA.

The present invention will be further described with reference to the following figures and examples; however, it is to be understood that the present invention is not limited to such figures and examples.

### Description of the drawings

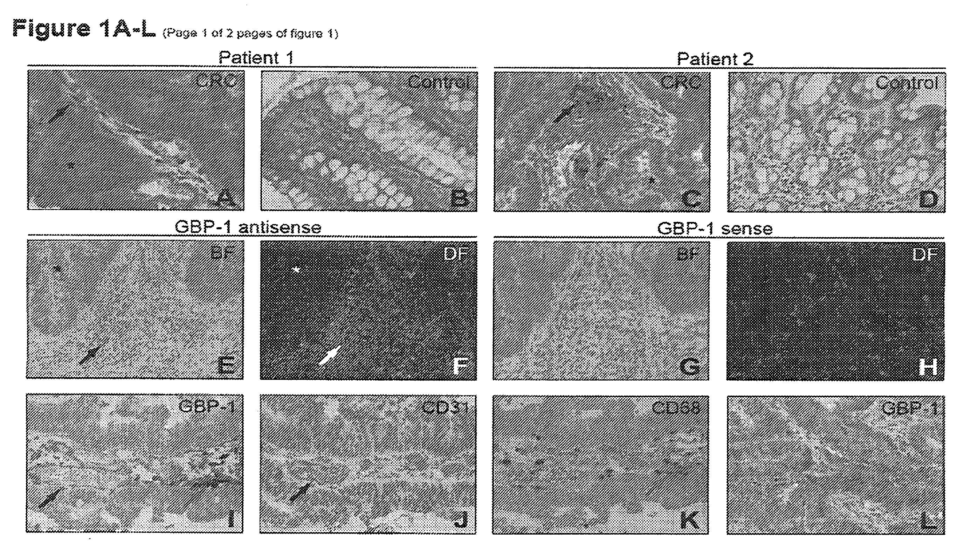

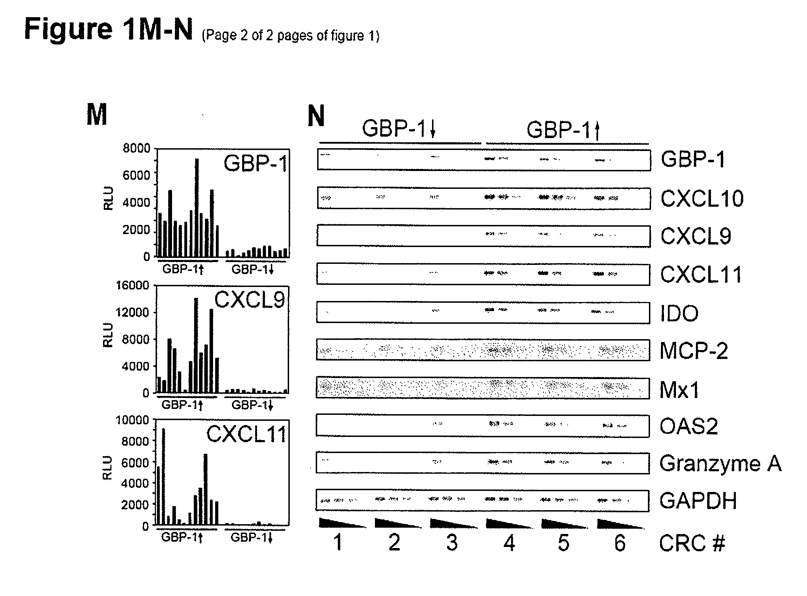

**Figure 1****. Coexpression of GBP-1 and interferon-induced angiostatic chemokines in colorectal carcinoma.** Immunohistochemical staining of GBP-1 in (**A, C**) CRC tissue and (**B, D**) healthy mucosa tissue of two representative patients. GBP-1-positive cells are indicated by an arrow, tumor cells are labeled by an asterisk. *In situ* hybridization of CRC tissue sections with ³⁵S-radiolabeled GBP-1 (**E, F**) antisense and (**G, H**) sense RNA strand hybridization probes. Prominent signals were obtained with the antisense hybridization probe (complementary to GBP-1 mRNA) in the stroma of CRC, both in the (**E**) bright field (BF, black grains) and (**F**) dark field (DF, white grains) exposure. (**G, H**) Control hybridization with the GBP-1 sense strand RNA probe did not show specific signals. Immunohistochemical staining of (**I**) GBP-1, (**J**) CD31 and (**K**) CD68 in consecutive sections of CRC. Corresponding tissue areas are indicated by arrows. (L) Example of a CRC tissue negative for GBP-1 in immunohistochemistry. Magnifications: (**A-D**) x850, (**E-L**) x530. (**M**) Normalized microarray signal intensities (relative light units: RLU) of GBP-1, CXCL9 and CXCL11 expression in GBP-1-positive (GBP-1↑, n=12) and GBP-1-negative CRC (GBP-1↓, n=12). The tumors are given at corresponding positions in each diagram. (**N**) Semi-quantitative RT-PCR of GBP-1 coregulated genes (CXCL10, CXCL9, CXCL11, IDO, MCP-2, Mx1, OAS2 and granzyme A) in three different GBP-1-positive (GBP-1↑) and GBP-1-negative (GBP-1↓) CRC. Decreasing amounts of cDNA (undiluted, 1/10, 1/100 and 1/1000) of the different tumors were subjected to each PCR. Amplification of GAPDH demonstrates that equal amounts of cDNA were used from each tumor.

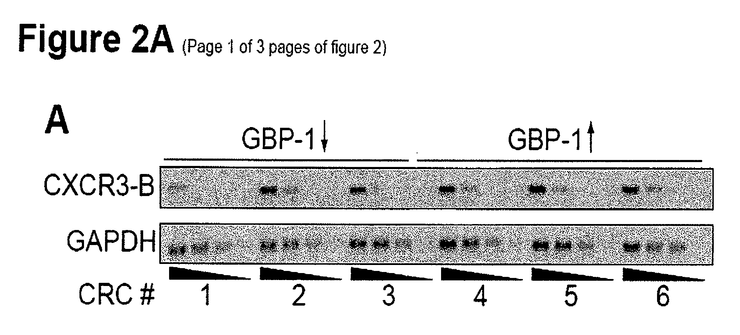

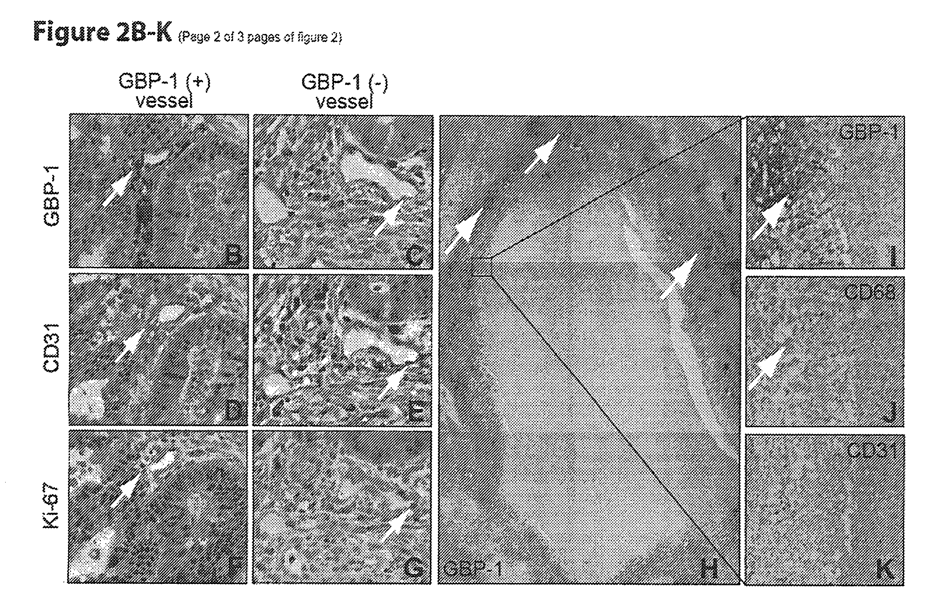

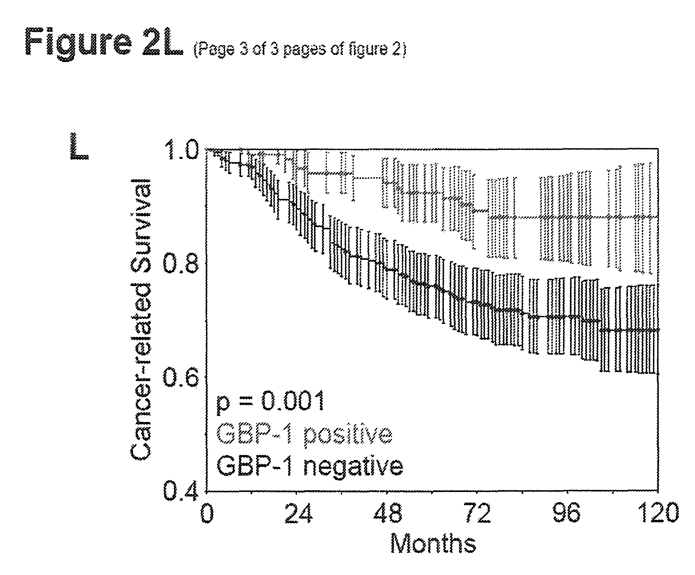

**Figure 2****. GBP-1 is associated with angiostasis and increased cancer-related 5-year survival in colorectal carcinoma.** (**A**) CXCR3-B expression was analyzed with semi-quantitative RT-PCR in three GBP-1-positive (GBP-1↑) and GBP-1-negative (GBP-1↓) CRC. CDNA was subjected in decreasing amounts (undiluted, 1/10,1/100 and 1/1000) to the PCR. Amplification of GAPDH demonstrates that equal amounts of cDNA of the different tumors were used. Immunohistochemical staining of (**B, C**) GBP-1, (**D**, **E**) CD31 and (**F, G**) Ki-67 (proliferation-associated antigen) on consecutive sections of GBP-1-positive (+) or negative (-) vessels. Corresponding cells are indicated by arrows. Immunohistochemical detection of (**H, I**) GBP-1, (**J**) CD68 and (**K**) CD31 in caseating tuberculosis. (**H**) Overview (GBP-1 positive cells, arrows) and (**I, J, K**) consecutive sections (corresponding cell indicated by arrows) of the field indicated in (**H**). Magnifications (**B-G**) x850, (**H**) x85, (**I-K**) x530. (**L**) Cancer-related 5-year survival of patients with GBP-1-positive (red, n = 124) and -negative colonic carcinoma (black, n = 264). The cancer-related survival is depicted by a Kaplan-Meier-Curve and 95% confidence intervals.

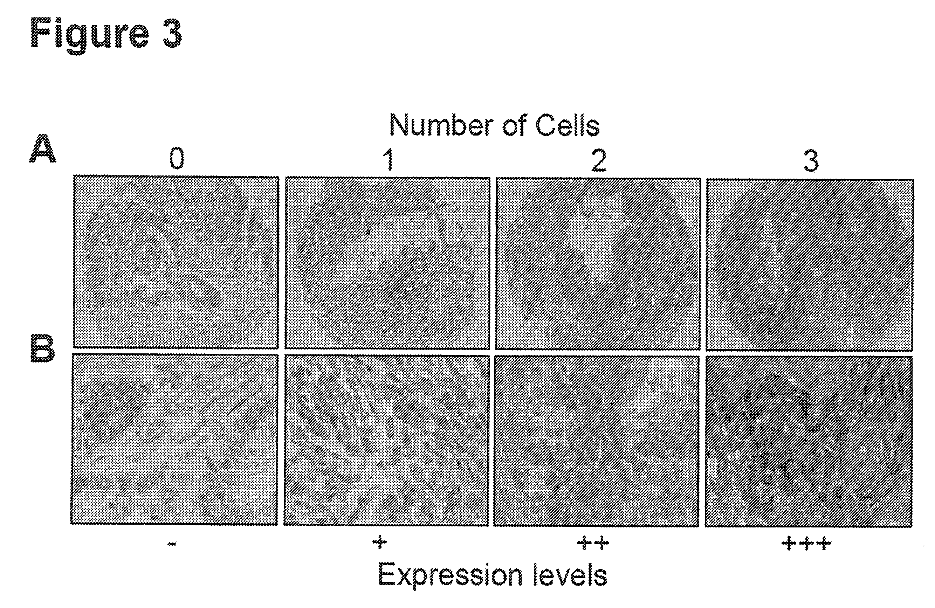

**Figure 3****. Quantification of GBP-1 staining in the CRC tissue array.** CRC tissue arrays were immunohistochemically stained for GBP-1 (brown). (**A**) Numbers of positive cells (0, negative; 1, < 50%; 2, ∼ 50%; 3, > 50%) and (**B**) GBP-1 expression levels (-, negative; +, weak; ++, middle; +++, high) were determined. Magnification x215.

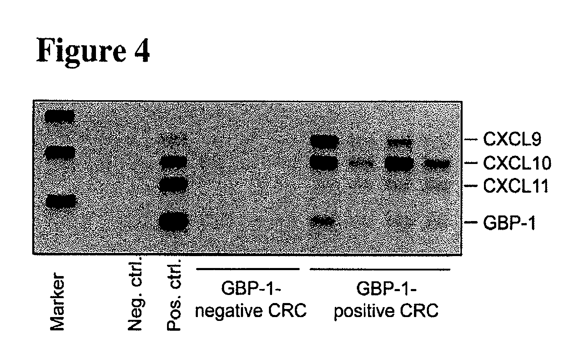

**Figure 4****. The anti-angiogenic chemokines CXCL9-11 are GBP-1-coregulated genes in the colorectal carcinoma (CRC).** A multiplex-RT-PCR for CXCL9-11 and GBP-1 using RNA from seven different colorectal carcinoma patients was performed. Patients were categorized as "GBP-1-negative" or "GBP-1-positive" according to immunohistochemistry results. As a negative (Neg. ctrl.) and positive control (Pos. ctrl.) RNA from unstimulated and IFN-γ-stimulated HUVEC, respectively was used in parallel.

### Examples

### Example 1: GBP-1 indicates an intrinsic angiostatic immune reaction in colorectal carcinoma

Robust expression of GBP-1 was detected in the desmoplastic stroma of colorectal carcinomas obtained from two different patients by immunohistochemistry (Fig. 1A, C, arrows). GBP-1 was not expressed in the tumor cells (Fig. 1A, C, asterisk) and in adjacent tumor free mucosa of the colon (Fig. 1B, D). These results were confirmed by *in situ* hybridization. With a GBP-1 mRNA specific probe strong signals were obtained in the tumor stroma exclusively (Fig. 1E, F, arrows, bright field [BF] and dark field [DF] of the same tissue section) but not in the tumor cell area (Fig. 1E, F, asterisk). No unspecific signals were obtained when the respective negative control probe was used (Fig. 1G, H; BF and DF of the same tissue section). Immunohistochemical staining of GBP-1, CD31 and CD68 in consecutive tumor sections demonstrated that GBP-1 (Fig. 1I) is expressed in endothelial cells (Fig. 1I, J, black arrows) and immune cells, most likely monocytes/macrophages (Fig. 1I, K, red arrows). In contrast, CRC obtained from three other patients did not express GBP-1 (Fig. 1L).

### Example 2: GBP-1 indicates an intrinsic angiostatic immune reaction in colorectal carcinoma

To characterize the GBP-1-associated micromilieu, 12 GBP-1-positive und 12 GBP-1-negative CRC of patients with closely matched clinical parameters (Table 1, lower panel) were identified by immunohistochemistry and subjected to a transcriptome analysis (HG-U133A, Affymetrix, 22,215 probe sets). Signals were normalized and listed according to their probability to reflect differential expression (p<0.05), significant signal intensity (>300 RLUs) and robust upregulation of expression (>4-fold) in GBP-1-positive tumors. 104 genes fulfilled these criteria (Table 4). Most of these genes were either well-known. IFN-induced genes, and/or encoded chemokines or immune reaction-associated genes (Table 4). Interestingly, the three major angiostatic chemokines (CXCL9, CXCL10, CXCL11: table 4, shaded) (Strieter et al., 2005b; Romagnani et al., 2004) were among the eight most strongly upregulated genes in GBP-1-positive tumors. Expression of angiogenic growth factors such as VEGF and basic fibroblast growth factor (bFGF) was not increased in GBP-1-positive CRC.

High reproducibility of the microarray analyses is demonstrated by the fact that within the groups of GBP-1-positive and -negative tumors highly reproducible results were obtained for each gene as shown exemplarily for GBP-1, CXCL9 and CXCL11 (Fig. 1M). In addition, semi-quantitative RT-PCR confirmed the microarray results showing that each of the three angiostatic chemokines (CXCL10, CXCL9, CXCL11) and of five additional IFN-γ-induced and/or immune reaction-associated genes [IFN-γ-inducible indoleamine 2,3-dioxygenase (IDO), monocyte chemotactic protein-2 (MCP-2), Mx1, 2'-5'-oligoadenylate synthetase-2 (OAS2) and granzyme A] were higher expressed in GBP-1-positive as compared to GBP-1-negative tumors (Fig. 1N).

An IFN-γ-dominated micromilieu characterized by the presence of the angiostatic chemokines has recently been described to regulate an intrinsic angiostatic immune reaction (IAR) (Strieter et al., 2005a; Strieter et al., 2006; Strieter et al., 2004; Strieter et al., 2005b). The antiangiogenic chemokines CXCL9-11 inhibit angiogenesis via the chemokine receptor CXCR3-B (Lasagni et al., 2003; Ehlert et al., 2004). RT-PCR showed that this receptor is constitutively expressed in both, GBP-1-positive and -negative CRC (Fig. 2A, CXCR3-B). Therefore, angiostasis can be induced in case CXCL9-11 are present. In addition, a negative correlation of GBP-1 expression and vessel proliferation supported the presence of angiostasis in GBP-1-positive tumors (Fig. 2B, D, F, arrows). Proliferating Ki-67-positive endothelial cells were exclusively detected in GBP-1-negative vessels but never in GBP-1-positive vessels (Fig. 2C, E, G, arrows; red nuclear Ki-67 staining indicates a proliferating endothelial cell). Finally, we challenged the concept that GBP-1 is associated with an intrinsic angiostatic immune reaction in a different disease. Caseating tuberculosis is the prototypic disease of IAR (Strieter et al., 2005a; Strieter et al., 2005b). This is most evident by the almost complete absence of blood vessels in the involved lung tissue. Immunohistochemical stainings of lung biopsies with caseating tuberculosis showed a robust GBP-1 signal (Fig. 2H, I, arrows). In agreement with the angiostatic conditions, endothelial cells were only rarely detected (Fig. 2K) and GBP-1-positive cells were predominantly macrophages (Fig. 2J, arrow).

In addition, 49 genes were identified, which were significantly increased in GBP-1-negative tumors (Table 5).

### Example 3. GBP-1 associated immunoangiostasis elongates survival of colorectal carcinoma patients

GBP-1 expression in UICC stage II-IV colonic carcinoma (n=388) was investigated by immunohistochemical tissue array technology (Tables 1 and 2). Nine different areas of each tumor were analyzed. Numbers of GBP-1-positive cells and expression levels were quantitatively determined (Fig. 3). GBP-1 was expressed in 32% of all tumors (Table 1, GBP-1 expression in the stroma) and was highly significant (p<0.001) associated with the early tumor stage (Table 2, see Stage and Regional Lymph Nodes). A considerably larger fraction of GBP-1-positive colonic carcinomas were UICC stage II (64.6%) and did not show lymph node metastasis (67.7% pN0) as compared to GBP-1-negative tumors (42.8% UICC II, 45.1% pN0). In contrast, GBP-1-negative tumors were more often in progressed UICC IV stage (11%) and showed metastasis in more than three lymph nodes (22.7% pN2) as compared to GBP-1-positive tumors (5.6% UICC IV, 12.1% pN2). Other clinical parameters such as primary tumor (pT-classification), histopathological grading or extramural venous invasion did not correlate significantly with GBP-1 expression (Table 2). The association with the UICC II stage was significant for all GBP-1-positive tumors, irrespectively of the absolute number of GBP-1-expressing cells and of GBP-1-expression level (Table 6, p value).

Interestingly, patients with GBP-1-positive colonic carcinoma had a highly significant (p<0.001) increased cancer-related 5-year survival rate of 16.2% in univariate analysis (Table 3, upper panel; Fig. 2L). Other well-established prognostic factors such as UICC stage, pT- and pN-status or extramural venous invasion did also correlate with increased survival confirming the representative value of this study group (Table 3). Most importantly, multivariate analysis showed that GBP-1 expression is an independent prognostic marker indicating a relative risk of cancer-related death of 0.5 as compared to colonic carcinoma patients that do not express GBP-1 (Table 3, lower panel).

### Material and Methods

### Clinical Samples

Affymetrix Array: After informed consent was obtained, 24 patients who underwent surgery for the first manifestation of CRC were included in the study. The investigation was carried out in accordance with the Helsinki declaration. Patients who underwent preoperative radiation or chemotherapy did not participate in the study (Table 1). Patients with familial CRC (familial adenomatous polyposis, hereditary nonpolyposis CRC) were excluded. Stage (UICC 2002), sex ratio, patient age, T-, N-, M-stage, histopathological grading and tumor site were used as conventional clinicopathological parameters (Table 1, lower panel).

Tissue Array: This study was based on the prospectively collected data of the Erlangen Registry of ColoRectal Carcinomas (ERCRC) from 1991 to 2001. 388 patients with the following inclusion criteria were selected: Solitary invasive colon carcinoma (invasion at least of the submucosa), localisation >16 cm from the anal verge, no appendix carcinoma; no other previous or synchronous malignant tumor, except squamous and basal cell carcinoma of the skin and carcinoma in situ of the cervix uteri; carcinoma not arisen in familial adenomatous polyposis, ulcerative colitis or Crohn's disease; treatment by colon resection with formal regional lymph node dissection at the Surgical Department of the University of Erlangen; residual tumor classification R0 (no residual tumor, clinical and pathohistological examination); UICC stage II-IV 2002 (UICC (2002) TNM classification of malignant tumors. 6th ed (Sobin LH, Wittekind Ch, eds). John Wiley & Sons, New York) (Table 1, upper panel). Patients who died postoperatively and patients with unknown tumor status (with respect to local and distant recurrence) at the end of the study (01.01.2006) were excluded. A total of nine punches from each of the 388 patients originating from tumor center (three punches), invasive front (three punches) and desmoplastic stroma in/adjacent to the tumor (three punches) were applied to the tissue array analysis. Median follow-up was 83 months (range 1-177). At the end of the study 88 patients (22.7%) had died of their colon carcinoma. Patient and tumor characteristics of the ERCRC patients are shown in Table 1, upper panel. Curatively resected distant metastases were located in the liver (n=29), distant lymph nodes (n=3), peritoneum (n=3), and others (n=3). The carcinomas were graded in accordance with the recommendations of the WHO using the categories low and high grade (Jass and Sobin 1989). With regard to venous invasion we distinguished between no or only intramural venous invasion (EVI negative [-]) and extramural venous invasion (EVI positive [+]). Emergency presentation was defined as the need for urgent surgery within 48 hours of admission (Soreide et al. 1997).

Caseating tuberculosis: Tissue sections of lung biopsies from six patients with the confirmed diagnosis caseating tuberculosis were obtained by the local pathology and areas including caseating granulomas were stained immunohistochemically.

### Immunohistochemical Staining

Staining for GBP-1, CD31, CD68 and Ki-67 was performed as previously described (Lubeseder-Martellato et al., 2002; Guenzi et al., 2001; Guenzi et al., 2003). The latter three antibodies were purchased from DAKO (Hamburg, Germany) and diluted as follows: CD31 (1:50), CD68 (1:200) and Ki-67 (1:300). Stained sections were evaluated by two independent persons. Differing results were evaluated by a third person and discussed until consensus was obtained.

### In Situ Hybridization

Biopsy specimens were processed as previously described (Stürzl et al., 1999; Sturzl et al., 1992). As a template for transcription of ³⁵S-labeled RNA sense/antisense hybridization probes full length GBP-1-encoding cDNA (M55542) was inserted into the pcDNA3.1 expression vector in sense/antisense orientation. T7 polymerase was used for *in vitro* transcription. After autoradiography sections were stained with haematoxylin and eosin and analyzed in the bright field (expression signals are black silver grains) and dark field (light scattering by silver grains produces white signals) with a Leica aristoplan microscope.

### RT-PCR Analysis

RT-PCR analysis was carried out by using the PCR primers (forward/reverse, 5'-3' orientation) for both, RT-PCR and multiplex RT-PCR: GBP-1 (M55542): ATGGCATCAGAGATCCACAT, GCTTATGGTACATGCCTTTC; CXCL10 (NM_001565.1): AAGGATGGACCACACAGAGG, TGGAAGATGGGAAAGGTGAG; CXCL9 (NM_002416.1): TCATCTTGCTGGTTCTGATTG, ACGAGAACGTTGAGATTTTCG; CXCL11 (AF030514.1): GCTATAGCCTTGGCTGTGATAT, GCCTTGCTTGCTTCGATTTGGG; IDO (M34455): GCAAATGCAAGAACGGGACACT, TCAGGGAGACCAGAGCTTTCACAC; MCP-2 (NM_005623): ATTTATTTTCCCCAACCTCC, ACAATGACATTTTGCCGTGA; Mx1 (NM_002462.2): TACAGCTGGCTCCTGAAGGA, CGGCTAACGGATAAGCAGAG; OAS2 (N_002535): TTAAATGATAATCCCAGCCC, AAGATTACTGGCCTCGCTGA; Granzyme A (NM_006144.2): ACCCTACATGGTCCTACTTAG, AAGTGACCCCTCGGAAAACA; CXCR3-B (AF469635): AGTTCCTGCCAGGCCTTTAC, CAGCAGAAAGAGGAGGCTGT; GAPDH: AGCCACATCGCTCAGAACAC, GAGGCATTGCTGATGATCTTG.

### Affymetrix GeneChip Analysis

Affymetrix GeneChip analysis was carried out as described previously (Croner et al., 2005a; Croner et al., 2005b; Croner et al., 2004). The whole microarray experiment design, setup and results are available through ArrayExpress (http://www.ebi.ac.uk/arrayexpressn using the access number E-MEXP-833.

### Statistical Analysis

Tissue array: The Kaplan-Meier method was used to calculate 5-year rates of cancer-related survival. An event was defined as "cancer-related death", i. e. death with recurrent locoregional or distant cancer. The 95% confidence intervals (95% CI) were calculated accordingly (Greenwood et al., 1926). Logrank test was used for comparisons of survival. A Cox regression analysis was performed to identify independent prognostic factors. All factors which were found significant in univariate survival analysis were introduced in the multivariate model. 2 patients were excluded because of missing data on extramural venous invasion (n=386). Chi-square test was used to compare frequencies. A p-value of less than 0.05 was considered to be statistically significant. Analyses were performed using SPSS software version 13 (SPSS Inc., Chicago, USA).

Affymetrix array: Raw data derived from GeneChips were normalized by "global scaling" using Affymetrix Microarray Suite, Data Mining Tool. Signals of the 12 GBP-1-positive and 12 GBP-1-negative CRCs, respectively, were averaged and upregulated genes selected according to p≤0.05, overall signal intensity >300 RLU and fold change >4.

### Tables

**Table 1. Clinical parameters of colonic carcinoma patients included in tissue array analysis (n=388) and of colorectal carcinoma patients included in gene chip analysis (n=24).**

| **TISSUE ARRAY ANALYSIS** | | | |
|---|---|---|---|
| | n | | % |
| Sex ratio (male/female) | 232/156 =1.5 | | |
| Age median / range (years) | 64/28-91 | | |
| **GBP-1 Expression In the Stroma** | | | |
| GBP-1-negative (-) | 264 | | 68.0 |
| GBP-1-positive (+) | 124 | | 32.0 |
| **Tumor Site** | | | |
| Sigmoid colon | 186 | | 47.9 |
| Descending colon | 16 | | 4.1 |
| **Splenic** flexure | 23 | | 5.9 |
| Transverse colon | 39 | | 10.1 |
| **Hepatic** flexure | 26 | | 6.7 |
| Ascending colon | 58 | | 14.9 |
| Cecum | 40 | | 10.3 |
| **Stage (UICC 2002)** | | | |
| II | 193 | | 49.7 |
| III | 159 | | 41.0 |
| IV | 36 | | 9.3 |
| **Primary Tumor** | | | |
| pT2 | 27 | | 7.0 |
| pT3 | 311 | | 80.2 |
| pT4 | 50 | | 12.9 |
| **Regional Lymph Nodes** | | | |
| pN0 | 203 | | 52.3 |
| pN1 | 110 | | 28.4 |
| | | | |
| pN2 | 75 | | 19.3 |
| **Histopathological Grading** | | | |
| Low grade (G1/G2) | 316 | | 81.4 |
| High grade (G3/G4) | 72 | | 18.6 |
| **Extramural Venous Invasion (EVI)** | | | |
| EVI (-) | 340 | | 87.6 |
| EVI (+) | 46 | | 11.9 |
| **Adjuvant Chemotherapy** | | | |
| No | 311 | | 80.2 |
| Yes | 77 | | 19.8 |
| **Emergency Presentation** | | | |
| No | 345 | | 88.9 |
| Yes | 43 | | 11.1 |

| **AFFYMETRIX GENE CHIP ANALYSIS** | | | |
|---|---|---|---|
| | **GBP-1-positive** | **GBP-1-negative** | **P value** |
| n | 12 | 12 | |
| Sex ratio (male/female) | 6/6 = 1 | 8*/3 = 2.6 | 0.265 |
| Age median / range (years) | 69.5/47-80 | 63* /46-75 | 0.453 |
| **Tumor Site** | | | 0.111 |
| Sigmoid colon | | 2 | |
| Rectum | 5 | 8 | |
| Descending colon | | 1 | |
| Splenic flexure | | 1 | |
| Transverse colon | 1 | | |
| Hepatic flexure | 1 | | |
| Ascending colon | 1 | | |
| Cecum | 4 | | |
| **Stage (UICC 2002)** | | | 0.459 |
| I 3 | | 2 | |
| II | 4 | 2 | |
| III | 5 | 8 | |
| **Primary Tumor** | | | 0.128 |
| pT1 | 1 | | |
| pT2 | 3 | 3 | |
| pT3 | 8 | 5 | |
| pT4 | | 4 | |
| **Regional Lymph Nodes** | | | 0.148 |
| pN0 | 7 | 4 | |
| pN1 | 5 | 5 | |
| pN2 | | 3 | |
| **Distant Metastasis** | | | |
| M0 | 12 | 12 | |
| **Histopathological Grading** | | | 0.132 |
| G2 | 11 | 8 | |
| G3 | 1 | 4 | |
| **Adjuvant chemotherapy (yes/no)** | 12/0 | 11/1 | 0.307 |

P value was assessed using Pearson's chi square test. *Gender and age of one patient was unknown.

**Table 2. GBP-1 expression is highly significant associated with UICC stage II/pN0-status of colonic carcinoma (n=388).**

| | **GBR-1 negative n°264** | **GBP-1 positive n°124** | **P-value** |
|---|---|---|---|
| **Stage (UICC 2002)** | | | <0.001 |
| II | 113 (42.8%) | 80 (64.6%) | |
| III | 122 (46.2%) | 37 (29.8%) | |
| IV | 29 (11%) | 7 (5.6%) | |
| **Primary Tumor** | | | 0.411 |
| pT2 | 16 (6.0%) | 11 (8.9%) | |
| pT3 | 211 (79.9%) | 100 (80.6%) | |
| pT4 | 37 (14.1%) | 13 (10.5%) | |
| **Regional Lymph Nodes** | | | <0.001 |
| pN0 | 119 (45.1%) | 84 (67.7%) | |
| pN1 | 85 (32.2%) | 25 (20.2%) | |
| pN2 | 60 (22.7%) | 15 (12.1%) | |
| **Histopathological Grading** | | | 0.264 |
| Low grade (G1/G2) | 219 (83.0%) | 97 (78.2%) | |
| High grade (G3/G4) | 45 (17.0%) | 27 (21.8%) | |
| **Extramural Venous Invasion** | | | 0.056 |
| EVI (-) | 226* (85.6%) | 114* (91.9%) | |
| BVI (+) | 37* (14.0%) | 9* (7.2%) | |

| | | | |
|---|---|---|---|
| *Extramural venous invasion status of two patients was unknown. P value was determined by Pearson's chi square test. | | | |

**Table 3. Cancer-related 5-year survival is highly significant increased in GBP-1-positive colonic carcinoma patients and indicates a significantly decreased relative risk of cancer-related death (n=388).**

| **UNIVARIATE ANALYSIS** | **n °** | **5 year cancer-related survival (%)** | **95% CI** | **P-value** |
|---|---|---|---|---|
| **All Patients** | 399 | 81.1 | 77.2-85.0 | |
| **GBP-1 Expression in** the **Stroma** | | | | <0.001 |
| GBP-1 neg. (-) | 264 | 76.0 | 70.7-81.3 | |
| GBP-1 pos. (+) | 124 | 92.2 | 87.3-97.1 | |
| **Stage (UICC 2002)** | | | | <0.001 |
| II | 193 | 91.6 | 87.5-95.7 | |
| III | 159 | 74.2 | 67.3-81.1 | |
| IV | 36 | 57.3 | 40.8-73.8 | |
| **Primary Tumor** | | | | 0.005 |
| pT2 | 27 | 96.2 | 88.8-100 | |
| pT3 | 311 | 82.3 | 78.0-86.6 | |
| pT4 | 50 | 64.8 | 51.3-78.3 | |
| **Regional Lymph Nodes** | | | | <0.001 |
| pN0 | 203 | 90.0 | 85.7-94.3 | |
| pN1 | 110 | 86.2 | 79.7-92.7 | |
| pN2 | 75 | 49.1 | 37.3-60.9 | |
| **Histopathological Grading** | | | | 0.134 |
| Low grade (G1/G2) | 316 | 82.4 | 78.1-86.7 | |
| High grade (G3/G4) | 72 | 75.2 | 65.0-85.4 | |
| **Extramural** Venous **Invasion** | | | | <0.001 |
| EVI (-) | 340* | 85.8 | 82.1-89.5 | |
| EVI (+) | 46* | 47.6 | 32.7-62.5 | |
| **Adjuvant Chemotherapy** | | | | 0.207 |
| No | 311 | 82.4 | 78.1-86.7 | |
| Yes | 77 | 75.7 | 65.9-85.5 | |
| **Emergency Presentation** | | | | <0.001 |
| No | 345 | 83.7 | 79.8-87.6 | |
| Yes | 43 | 57.8 | 42.1-73.5 | |

| **MULTIVARIATE ANALYSIS** | **n°** | **Relative Risk** | **95% CI** | **P-value** |
|---|---|---|---|---|
| **GBP-1 Expression** in **the Stroma** | | | | |
| GBP-1 negative (-) | 263 | 1.0 | | |
| GBP-1 positive (+) | 123 | 0.5 | 0.3-0.9 | 0.032 |
| Stage (UICC 2002) | | | | |
| Stage II | 193 | 1.0 | | |
| Stage III | 157 | 2.5 | 1.5-4.2 | 0.001 |
| Stage IV | 36 | 4.3 | 2.2-8.3 | <0.001 |
| **Extramural Venous Invasion** | | | | |
| EVI (-) | 340* | 1.0 | | |
| EVI (+) | 46* | 2.7 | 1.7-4.4 | <0.001 |
| **Emergency Presentation** | | | | |
| No | 344 | 1.0 | | |
| Yes | 42 | 2.1 | 12-3.7 | 0.008 |

| | | | | |
|---|---|---|---|---|
| *Extramural venous invasion status of two patients was unknown. Accordingly, the cancer-related 5-year survival of 388 patients and the relative risk of 386 patients, respectively were analyzed. 95% confidence intervals (95%-CI) and p values as determined by univariate analysis (upper) and multivariate analysis (lower) are given in relation to clinical parameters. | | | | |

**Table 4. GBP-1-positive colorectal carcinomas (n=12) were compared with GBP-1-negative CRCs (n=12) by transcriptome analysis.**

| Seq. No. | Fold change | P value | Accession Number | Gene | Group |
|---|---|---|---|---|---|
| 1 | 25.52 | 0 | AF030514.1 | Homo sapiens interferon stimulated T-cell alpha chemoattractant (CXCL11) | IFN, CC |
| 2 | 17.74 | 0.004 | D87021 | Homo sapiens immunoglobulin lambda gene locus DNA | IR |
| 3 | 16.79 | 0 | AF002985.1 | Homo sapiens putative alpha chemokine (H174) | CC |
| 4 | 14.36 | 0 | NM_002416.1 | Homo sapiens monokine induced by gamma interferon (CXCL9) | IFN, CC |
| 5 | 14.34 | 0 | NM_005601.1 | Homo sapiens natural killer cell group 7 sequence (NKG7) | IR |
| 6 | 13.8 | 0.001 | M24669.1 | Human Ig rearranged H-chain V-region mRNA (C-D-JH6) | IR |
| 7 | 13.21 | 0.002 | M24668.1 | Human Ig rearranged H-chain V-region mRNA (C-D-JH4) | IR |
| 8 | 13.01 | 0 | NM_001565.1 | Homo sapiens small inducible cytokine subfamily B (Cys-X-Cys), member 10 (CXCL10) | IFN, CC |
| 9 | 12.8 | 0 | NM_006820.1 | Homo sapiens interferon-induced protein 44-like (IFI44L) | IFN |
| 10 | 12.13 | 0.003 | BG482805 | Homo sapiens rearranged gene for kappa immunoglobulin subgroup V kappa IV | IR |
| 11 | 12.07 | 0.001 | L34164.1 | Human Ig rearranged mu-chain gene VH3-D21 10-JH2 | IR |
| 12 | 10.81 | 0.002 | AV698647 | Homo sapiens immunoglobulin lambda joining 3 | IR |
| 13 | 10.77 | 0 | L14458.1 | Human Ig rearranged kappa-chain gene V-J-region | IR |
| 14 | 10.7 | 0 | NM_006419.1 | Homo sapiens small inducible cytokine B subfamily, member 13 (SCYB13, CXCL13) | CC |
| 15 | 10.53 | 0.003 | L23518.1 | Human Ig rearranged gamma-chain, V-DXP1-JH4b | IR |
| 16 | 10.26 | 0.005 | U80139 | Human immunoglobulin heavy chain variable region (V4-4) gene | IR |
| 17 | 10.12 | 0.001 | L23516.1 | Human Ig rearranged gamma-chain, V-DXP4-JH6c | IR |
| 18 | 9.84 | 0.001 | AJ408433 | Homo sapiens partial IGKV gene for immunoglobulin kappa chain variable region, clone 38 | IR |
| 19 | 9.65 | 0.003 | M24670.1 | Human Ig rearranged H-chain V-region mRNA (C-D-JH6) | IR |
| 20 | 9.07 | 0.005 | AF234255.1 | Homo sapiens clone KM36 immunoglobulin light chain IR variable region | |
| 21 | 8.92 | 0 | BG540628 | Human active IgK chain from GM 607, V-kappa-2 region | IR |
| 22 | 8.88 | 0.007 | D84143.1 | Human immunoglobulin (mAb59) light chain V region | IR |
| 23 | 8.79 | 0.002 | M85256.1 | Homo sapiens immunoglobulin kappa-chain VK-1 (IgK) | IR |
| 24 | 8.73 | 0.002 | AJ275408 | Homo sapiens partial IGVH3 gene for immunoglobulin heavy chain V region, case 1, cell Mo VI 162 | IR |
| 25 | 8.58 | 0 | M21121 | Human T cell-specific protein (RANTES) | CC |
| 26 | 8.51 | 0.001 | M34455.1 | Human interferon-gamma-inducible indoleamine 2,3-dioxygenase (IDO) | IFN |
| 27 | 8.5 | 0.001 | X51887 | Human V108 gene encoding an immunoglobulin kappa orphon | IR |
| 28 | 8.07 | 0.004 | AJ275397 | Homo sapiens partial IGVH1 gene for immunoglobulin heavy chain V region, case 1, cell Mo V 94 | IR |
| 29 | 7.71 | 0.002 | AB035175 | Homo sapiens IgH VH gene for immunoglobulin heavy | IR |
| | | | | chain | |
| 30 | 7.7 | 0.001 | L14457.1 | Human Ig rearranged kappa-chain gene V-J-region | IR |
| 31 | 7.65 | 0.003 | AF103529.1 | Homo sapiens isolate donor N clone N88K immunoglobulin kappa light chain variable region light chain variable region | IR |
| 32 | 7.46 | 0.024 | AF047245.1 | Homo sapiens clone bsmneg3-t7 immunoglobulin lambda light chain VJ region, (IGL) | IR |
| 33 | 7.45 | 0.005 | NM_021181.2 | Homo sapiens SLAM family member 7 (SLAMF7) | IR |
| 34 | 7.44 | 0.001 | AJ275469 | Homo sapiens partial IGVH3 gene for immunoglobulin heavy chain V region, case 2, cell E 172 | IR |
| 35 | 7.35 | 0.001 | H53689 | Homo sapiens clone ASPBLL54 immunoglobulin lambda light chain VJ region | IR |
| 36 | 7.29 | 0.001 | AJ249377.1 | Homo sapiens partial mRNA for human Ig lambda light chain variable region, clone MB91 | IR |
| 37 | 7.2 | 0.003 | M16768.1 | Human T-cell receptor gamma chain VJCI-CII-CIII region | IR |
| 38 | 7.11 | 0.001 | M85276 | Homo sapiens NKG5 gene | other |
| 39 | 6.92 | 0.009 | M87268.1 | Human IgM VDJ-region | IR |
| 40 | 6.82 | 0.001 | Y13710 | Homo sapiens mRNA for alternative activated macrophage specific CC chemokine 1 | CC |
| **41** | **6.73** | **0** | **BC002666.1** | **Homo sapiens, guanylate binding protein 1, interferon-inducible, 67kD** | **IFN** |
| 42 | 6.73 | 0.001 | AW408194 | Homo sapiens immunoglobulin kappa variable 1-13 | IR |
| 43 | 6.72 | 0 | NM_000579.1 | Homo sapiens chemokine (C-C motif) receptor 5 (CCR5) | CC |
| 44 | 6.69 | 0.008 | BF002659 | Myosin-reactive immunoglobulin heavy chain variable region | IR |
| 45 | 6.47 | 0 | NM_004335.2 | Homo sapiens bone marrow stromal cell antigen 2 (BST2) | IR |
| 46 | 6.43 | 0.005 | AF043583.1 | Homo sapiens clone ASMnegl-b3 immunoglobulin lambda chain VJ region, (IGL) | IR |
| 47 | 6.36 | 0 | NM_004585.2 | Homo sapiens retinoic acid receptor responder (tazarotene induced) 3 (RARRPS3) | other |
| 48 | 6.31 | 0.003 | X79782.1 | H.sapiens (T1.1) mRNA for IG lambda light chain. | IR |
| 49 | 6.22 | 0.004 | X93006.1 | H.sapiens mRNA for IgG lambda light chain V-J-C region (clone Tgl11) | IR |
| 50 | 6.19 | 0.002 | NM_006433.2 | Homo sapiens granulysin (GNLY), transcript variant NKG5 | IR |
| 51 | 6.17 | 0.001 | AA680302 | Homo sapiens immunoglobulin lambda locus | IR |
| 52 | 6.03 | 0.001 | BG536224 | Human kappa-immunoglobulin germline pseudogene (Chr22.4) variable region (subgroup V kappa II) | IR |
| 53 | 5.81 | 0.015 | L23519.1 | Human Ig rearranged gamma-chain, V-DK4-JH4b | IR |
| 54 | 5.7 | 0 | AI984980 | small inducible cytokine subfamily A, member 8 (monocyte chemotactic protein 2) (MCP-2) | CC |
| 55 | 5.69 | 0.002 | AB000221.1 | Homo sapiens mRNA for CC chemokine | CC |
| 56 | 5.65 | 0.005 | AJ239383.1 | Homo sapiens mRNA for immunoglobulin heavy chain variable region, ID 31 | IR |
| 57 | 5.63 | 0.001 | U92706 | Homo sapiens mRNA for single-chain antibody | IR |
| 58 | 5.6 | 0.002 | AB001733.1 | Homo sapiens mRNA for single-chain antibody | IR |
| 59 | 5.52 | 0 | NM_006144.2 | Homo sapiens granzyme A (granzyme 1, cytotoxic T-lymphocyte-associated serine esterase 3) GZMA | IR |
| 60 | 5.45 | 0.003 | AW404894 | Homo sapiens partial IGKV gene for immunoglobulin kappa chain variable region, clone 30 | IR |
| 61 | 5.43 | 0.001 | NM_001548.1 | Homo sapiens interferon-induced protein with tetratricopeptide repeats 1 (IFIT1) | IFN |
| 62 | 5.42 | 0.001 | NM_000570.1 | Homo sapiens Fc fragment of IgG, low affinity IIIb, receptor for (CD16) (FCGR3B) | IR |
| 63 | 5.35 | 0.001 | AF103530.1 | Homo sapiens isolate donor N clone N8K immunoglobulin kappa light chain variable region | IR |
| 64 | 5.33 | 0001 | M20812 | Human kappa-immunoglobulin germline pseudogene (cos118) variable region (subgroup V kappa I) | IR |
| 65 | 5.25 | 0 | NM_002535.1 | Homo sapiens 2'-5'-oligoadenylate synthetase 2 (OAS2), transcript variant 2 | IFN |
| 66 | 5.08 | 0 | AI337069 | Homo sapiens cDNA clone IMAGE 2009047 | other |
| 67 | 5.04 | 0.001 | M30894.1 | Human T-cell receptor Ti rearranged gamma-chain mRNA V-J-C region | IR |
| 68 | 5 | 0.001 | BG340548 | Human rearranged immunoglobulin heavy chain | IR |
| 69 | 4.98 | 0.001 | BG485135 | immunoglobulin kappa variable 3D-15 | IR |
| 70 | 4.98 | 0.001 | AB014341.1 | Homo sapiens mRNA for VEGF single chain antibody | IR |
| 71 | 4.93 | 0.001 | AF043179.1 | Homo sapiens T cell receptor beta chain (TCRBV13S1-TCRBJ2S 1) | IR |
| 72 | 4,87 | 0.001 | M87790.1 | Human (hybridoma H210) anti-hepatitis A immunoglobulin lambda chain variable region, constant region, complementarity-determining regions | IR |
| 73 | 4.79 | 0 | AI768628 | Homo sapiens IMAGE clone similar to: chloride intracellular channel 2 | other |
| 74 | 4.69 | 0.001 | M27487.1 | Homo sapiens MHC class II DPw3-alpha-1 chain | IR |
| 75 | 4.54 | 0.013 | L14456.1 | Human Ig rearranged mu-chain gene V-N-D-N-J-region | IR |
| 76 | 4.51 | 0 | NM_006332.1 | Homo sapiens interferon, gamma-inducible protein 30 (IFI30) | IFN |
| 77 | 4.47 | 0 | NK_017523.1 | Homo sapiens XIAP associated factor-1 (BIRC4BP) | other |
| 78 | 4.41 | 0.007 | BG397856 | major histocompatibility complex, class II, DQ alpha 1 | IR |
| 79 | 4.4 | 0 | BC002704.1 | Homo sapiens, Similar to signal transducer and activator of transcription 1,9 kD | IFN |
| 80 | 4.39 | 0.001 | NM_022873.1 | Homo sapiens interferon, alpha-inducible protein (clone IFI-6-16) (G1P3), transcript variant 3 | IFN |
| 81 | 4.36 | 0 | NM_002462.1 | Homo sapiens myxovirus (influenza) resistance 1, homolog of murine (interferon-inducible protein p78) (MX1) | IFN |
| 82 | 4.33 | 0 | M87789.1 | Human (hybridoma H210) anti-hepatitis A IgG variable region, constant region, complementarity-determining regions | IR |
| 83 | 4.31 | 0.002 | X57812.1 | Human rearranged immunoglobulin lambda light chain | IR |
| 84 | 4.29 | 0 | NM_006398.1 | Homo sapiens diubiquitin (UBD) | other |
| 85 | 4.27 | 0 | NM_002838.1 | Homo sapiens protein tyrosine phosphatase, receptor type, C (PTPRC) | other |
| 86 | 4.27 | 0.001 | NM_001803.1 | Homo sapiens CD52 antigen (CAMPATH-1 antigen) (CD52) | IR |
| 87 | 4.25 | 0 | NM_001775.1 | Homo sapiens CD38 antigen (p45) (CD38) | IR |
| 88 | 4.25 | 0.002 | M80927.1 | Human glycoprotein mRNA | other |
| 89 | 4.21 | 0.007 | NM_006498.1 | Homo sapiens lectin, galactoside-binding, soluble, 2 (galectin 2) (LGALS2) | IR |
| 90 | 4.19 | 0 | NM_005101.1 | Homo sapiens interferon-alpha inducile (clone IFI-ISK) (G1P2) | IFN |
| 91 | 4.19 | 0 | NM_006417.1 | Homo sapiens interferon-induced, protein 44 (IFI 44) | IFN |
| 92 | 4.17 | 0.001 | BC000879.1 | Homo sapiens, Similar to kynureninase (L-kynurenine hydrolase), clone MGC:5080 | other |
| 93 | 4.14 | 0.001 | M60334.1 | Human MHC class II HLA-DR-alpha | IR |
| 94 | 4.13 | 0.003 | NM_004503.1 | Homo sapiens homeo box C6 (HOXC6) | other |
| 95 | 4.09 | 0.001 | NM_012307.1 | Homo sapiens erythrocyte membrane protein band 4.1-like 3 (EPB41L3) | other |
| 96 | 4.08 | 0 | NM_004244.1 | Homo sapiens CD163 antigen (CD 163) | IR |
| 97 | 4.08 | 0 | NM_002201.2 | Homo sapiens interferon stimulated gene (20kD) (ISG20) | IFN |
| 98 | 4.07 | 0 | A1809341 | IMAGE clone similar to: protein tyrosine phosphatase, receptor type, C (PTPRC) | other |
| 99 | 4.07 | 0.002 | M60333.1 | Human MHC class II HLA-DRA | IFN |
| 100 | 4.05 | 0.003 | NM_001623.2 | Human allograft-inflammatory factor-1 (AIF-1) | IFN |
| 101 | 4.04 | 0 | NM_017631.1 | hypothetical protein FLJ20035 | other |
| 102 | 4.02 | 0 | NM_002121.1 | Homo sapiens major histocompatibility complex, class II, DPbeta 1 | IR |
| 103 | 4.02 | 0.002 | AL022324 | Human DNA sequence from clone CTA-246H3 on chromosome 22 Contains the gene for IGLL 1 (immunoglobulin lambda-like polypeptide 1, pre-B-cell specific) | IR |
| 104 | 4.01 | 0.015 | M17955.1 | Human MHC class II HLA-DQ-beta | IR |
| 105 | | | Gi: 48146240 | Homo sapiens, guanylate binding protein 2, | |
| 106 | | | Gi: 24308156 | Homo sapiens, guanylate binding protein 3, | |
| 107 | | | Gi: 15558942 | Homo sapiens, guanylate binding protein 4, | |
| 108 | | | Gi: 31377630 | Homo sapiens, guanylate binding protein 5, | |

Genes estimated to be significantly increased in GBP-1-positive CRC are given in the table by fold change increase. Genes were functionally grouped into IFN-induced genes (IFN), chemokines (CC), immune reaction-associated genes (IR) and others. P value was assessed by Mann-Whitney-U-test. Gene names and the corresponding gene bank number are given. The three antiangiogenic chemokines and GBP-1 are shaded.

**Table 5. Genes downregulated in GBP-1-positive CRC**

| **Seq.No.** | **Average GBP-1-positive CRC** | **Average GBP-1-negative CRC** | **Fold increase** | **p value of differential expression** | **Accession number GB** | **Description** |
|---|---|---|---|---|---|---|
| 109 | 79.12 | 1470.02 | 18,58 | 0.008 | NM_000439.2 | Homo sapiens proprotein convertase subtilisin kexin type 1 (PCSKl) |
| 110 | 45.22 | 472.22 | 10.44 | 0.006 | NM_004626.1 | Homo sapiens wingless-type MMTV integration site family. member 11 (WNT11) |
| 111 | 175.88 | 795.85 | 4.52 | 0.038 | NM_001853.1 | Homo sapiens collagen. type IX alpha 3 (COL9A3) |
| 112 | 309.95 | 1387.91 | 4.48 | 0.033 | NM_007197.1 | Homo sapiens frizzled (Drosophila) homolog 10 (FZD10) |
| 113 | 186.97 | 722.4 | 3.86 | 0.05 | NM_007191.1 | Homo sapiens Wnt inhibitory factor-1 (WIF-1) |
| 114 | 94.52 | 348.81 | 3.69 | 0.003 | AF202063.1 | Homo sapiens oroblast growth factor receptor 4. soluble-form spliee varient (FGFR4) |
| 115 | 1435.76 | 5248.49 | 3-66 | 0.008 | NM_001823.1 | Homo sapiens creatine kinase brain (CKB) (CIG) |
| 116 | 130.63 | 447.83 | 3.43 | 0.021 | NM_004796.1 | Homo sapiens xin 3 (NRXN3) |
| 117 | 159.13 | 526.83 | 3.31 | 0.002 | NM_004636.1 | Homo sapiens sema domain immunoglobulin domain (Ig), short basic domain secreted (semaphorin) 3B (SEMA3B) |
| 118 | 204.43 | 663.17 | 3.24 | 0.001 | NM_012410.1 | Homo sapiens type I transmembrane receptor (seizure-related protein) (PSK-1) |
| 119 | 1078.19 | 3477.69 | 3.23 | 0.043 | NM_005588.1 | Homo sapiens meprin A. alpha (PABA peptide hydrolase) (MEP1A) |
| 120 | 285.67 | 837.78 | 2.93 | 0.043 | NM_006198.1 | Homo sapiens Purkinje cell protein 4 (PCP4) |
| 121 | 183.31 | 534.82 | 2.91 | 0.021 | AF195953 | Homo sapiens membrane-bound aminopepridase P(XNPEP2) |
| 122 | 112.07 | 322.61 | 2.88 | 0.033 | AW770748 | imprinted in Prader-Willi syndrome |
| 123 | 332.18 | 893.32 | 2.7 | 0.002 | AB002360.1 | Human mRNA for KIAA0362 gene |
| 124 | 5098.08 | 13469.6 | 2.64 | 0.033 | D13889.1 | Human mRNA for Id-1H |
| 125 | 1745.44 | 4355,77 | 2.52 | 0.003 | NM_003212.1 | Homo sapiens teratocarcinoma-derived growth factor 1 (TDGF1) |
| 126 | 137.29 | 344.38 | 2.51 | 0.021 | NM_001808.1 | Homo sapiens carboxyl ester lipase-like (bike alt-stimulated lipese-like) (CELL) |
| 127 | 269.58 | 670.96 | 2.49 | 0 | NM_017797.1 | Homo sapiens BTB (POZ) domain containing 2 (BTBD2) |
| 128 | 472.86 | 1153.52 | 2.44 | 0.004 | NM_015392.1 | Homo sapiens neural proliferation differentiation and control I (NPDC1) |
| 129 | 156.47 | 372.88 | 2.38 | 0.009 | AL531533 | branched chain keto said dehydrogenase E. beta polypeptide (maple syrup urine disense) |
| 130 | 864.83 | 2043.48 | 2.36 | 0.043 | NM_001926.2 | Homo sapiens defensin, alpha 6. Paneth cell-specific (DEFA6) |
| 131 | 3010.33 | 6976.21 | 2.32 | 0.002 | NM_018487.1 | Homo sapiens hepatecellular carcinoma-associated antigen 112 (HCA112) |
| 132 | 138.36 | 319.83 | 2.31 | 0.001 | NM_000724.1 | Homo sapiens calcium channel, voltage-dependent beta 2 subunit (CACNB2) |
| 133 | 176.45 | 406.52 | 2.3 | 0.008 | NM_021924.1 | Homo sapiens mucin and cadherin-like (MUCDHL) |
| 134 | 742.42 | 1703.29 | 2.29 | 0.007 | NM_002591.1 | Homo sapiens phosphoenolpyrivate carboxykinase 1 (soluble) (PCK1) |
| 135 | 987.26 | 2255.8 | 2.28 | 0.006 | Al049593 | phosphoinositide-specific phospholipase C-beta I/DEF |
| 136 | 397.75 | 902.54 | 2.27 | 0,018 | NM_025081.1 | Homo sapiens KIAAI305 protein (KIAA1305) |
| 137 | 230.82 | 521.74 | 2.26 | 0.021 | NM_013358.1 | Homo sapiens peptidylargimine deiminase type I (hPAD-colony10) |
| 138 | 2061.12 | 4619.07 | 2.24 | 0.003 | I20317.1 | Homo sapiens tyrosine protein kinase (CAK) gene |
| 139 | 257.46 | 576.21 | 2.24 | 0.053 | MM_000015.1 | Hamo sapiens N-acetyltransferase 2 (arylamine N-acetyltransferase) (NAT2) |
| 140 | 176.29 | 393-54 | 2.23 | 0.038 | X17406.1 | Human mRNA for cartilage specific proteglycan |
| 141 | 169.29 | 376.37 | 2.22 | 0.021 | NM_005060.1 | Homo sapiens RAR-ralated orphan receptor C (RORC) |
| 142 | 249.42 | 548.12 | 2.2 | 0.009 | NM_016202.1 | Homo sapiens LDL induced EC protein (LOC51157) |
| 143 | 363.79 | 788.76 | 2.17 | 0.009 | U35622.2 | Homo sapiens EWS protein E1A enhancer binding protein chimera |
| 144 | 583.47 | 1257.57 | 2.16 | 0.002 | AB038783.1 | Homo sapiens MUC3B mRNA for intertinal mucin |
| 145 | 239.74 | 506.5 | 2.11 | 0.001 | NM_004658.1 | Homo sapiens RAS protein activator like 1 (GAP1 like)RASAL1) |
| 146 | 390.65 | 822.6 | 2.11 | 0.038 | NM_005975.1 | Homo sapiens PTK6 protein tyrosine kinase 6 (PTK6) |
| 147 | 144.03 | 302.12 | 2.1 | 0.038 | NM_000504.2 | Homo sapiens coagulation factor X (F10) |
| 148 | 523.33 | 1094.1 | 2.09 | 0.008 | NM_000196.1 | Homo sapiens hyroxysteroid (11- beta) dehydrogenase 2 (HSB11B2) |
| 149 | 2572.06 | 5352.47 | 2.08 | 0.008 | NM_001038.1 | Homo sapiens sodium channel nonvoltage-gated 1 alpha (SCN1A) 2 |
| 150 | 2141.68 | 4420.33 | 2.06 | 0.002 | NM_001954.2 | Homo sapiens discoidin domain receptor family member 1 (DDR1). transcript variant |
| 151 | 2173.38 | 4478,25 | 2.06 | 0.021 | NM_003915.1 | Homo sapiens copine I (CPNE1) |
| 152 | 573.38 | 1167.21 | 2.04 | 0.001 | U51096.1 | human homeobox protein Cdx2 |
| 153 | 8.537.94 | 17329.82 | 2.03 | 0.005 | BE542815 | general transcription factor IIIA |
| 154 | 456.18 | 925.45 | 2.03 | 0.038 | NK_004624.1 | Homo sapiens vasoactive intestinal peptide receptor 1 (VIPR1) |
| 155 | 691.82 | 1399.03 | 2.02 | 0.043 | NM_002705.1 | Homo sapiens periplakin (PPL) |
| 156 | 217.06 | 437.27 | 2.01 | 0.013 | NM_016339.1 | Homo sapiens Link guamine nucleotide exchange factor II (LOC51195) |
| 157 | 892.73 | 1783.97 | 2 | 0.011 | NM_005766.1 | Homo sapiens FERM, RhoGEF (ARHGEF) and pleckstrin domain protein 1 (chrondrocyte-derived) (FARP1) |

**Table 6. The association of GBP-1 expression with UICC II stage/pN0 status is independent of the absolute number of GBP-1-positive cells and GBP-1 expression level.**

| | | **GBP-1: Number of Cells** | | | **P value** |
|---|---|---|---|---|---|
| | **0** | **1** | **2** | **3** | |
| **UICC stage** | | | | | |
| II | 122 (43.4%) | 37 (61.7%) | 28 (60.9%) | 20 (80%) | 0.001 |
| III | 129 (45.9%) | 22 (36.7%) | 14 (30.4%) | 3 (12%) | |
| IV | 30 (10.7%) | 1 (1.7%) | 4 (8.7%) | 2 (8%) | |
| **Pathologic** | | | | | |
| **Lymph Node** | | | | | |
| **Status** | | | | | |
| pN0 | 128 (45.6%) | 38 (63.3%) | 30 (65.2%) | 21 (84%) | 0.002 |
| pN1 | 91 (32.4%) | 13 (21.7%) | 10 (21.7%) | 3 (12%) | |
| pN2 | 62 (22.1%) | 9 (15%) | 6 (13%) | 1 (4%) | |

| | | **GBP-1: Expression Level** | | | **P value** |
|---|---|---|---|---|---|
| | - | + | ++ | +++ | |
| **UICC stage** | | | | | |
| II | 122 (43.4%) | 39 (62.9%) | 39 (66.1%) | 7 (70%) | 0.002 |
| III | 129 (45.9%) | 20 (32.3%) | 18 (30.5%) | 1 (10%) | |
| IV | 30 (10.7%) | 3 (4.8%) | 2 (3.4%) | 2 (20%) | |
| **Pathologic** | | | | | |
| **Lymph Node** | | | | | |
| **Status** | | | | | |
| pN0 | 128 (45.6%) | 41 (66.1%) | 39 (66.1%) | 9 (90%) | 0.002 |
| pN1 | 91 (32.4%) | 14 (22.6%) | 11 (18.6%) | 1 (10%) | |
| pN2 | 62 (22.1%) | 7 (11.3%) | 9 (15.3%) | - | |

CRC tissue arrays were immunohistochemically stained for GBP-1. Numbers of positive cells (0, negative; 1, < 50%; 2, ∼ 50%; 3, > 50%) and expression levels (-, negative; +, weak; ++, middle; +++, high) were determined. P values given were assessed by Pearsons's chi square test.

### Sequences:

CXCL9: Seq. No. 4; corresponds to SEQ ID NO: 1) nucleic acid sequence: amino acid sequence (corresponds to SEQ ID NO: 2):
CXCL10: (Seq. No. 8; corresponds to SEQ ID NO: 3) (corresponds to SEQ ID NO: 4)
CXCL11: (Seq. No. 1; corresponds to SEQ ID NO: 5) (corresponds to SEQ ID NO: 6)
GBP-1: (Seq. No. 41; corresponds to SEQ ID NO: 7) (corresponds to SEQ ID NO: 8)
GBP-2: (Seq. No. 105; corresponds to SEQ ID NO: 9) (corresponds to SEQ ID NO: 10)
GBP3: (Seq. No. 106; corresponds to SEQ ID NO: 11)
GBP-4: (Seq. No. 107; corresponds to SEQ ID NO: 12) (corresponds to SEQ ID NO: 13)
GBP-5: (Seq. No. 108; corresponds to SEQ ID NO: 14) (corresponds to SEQ ID NO: 15)

### References:

Baylin, S.B., and J.G. Herman. 2000. DNA hypermethylation in tumorigenesis: epigenetics joins genetics. Trends Genet. 16:168-74.
Bossi, P., G. Viale, A.K. Lee, R. Alfano, G. Coggi, and S. Bosari. 1995. Angiogenesis in colorectal tumors: microvessel quantitation in adenomas and carcinomas with clinicopathological correlations. Cancer Res. 55:5049-53.
Cheng YS, Colonno RJ and Yin FH. Interferon induction of fibroblast proteins with guanylate binding activity. J Biol Chem 1983; 258(12):7746-50.
Choi, H.J., M.S. Hyun, GJ. Jung, S.S. Kim, and S.H. Hong. 1998. Tumor angiogenesis as a prognostic predictor in colorectal carcinoma with special reference to mode of metastasis and recurrence. Oncology. 55:575-81.
Clevers H. At the crossroads of inflammation and cancer. Cell 2004; 118(6):671-4.
Cozzolino, F., M. Torcia, D. Aldinucci, M. Ziche, F. Almerigogna, D. Bani, and D.M. Stem. 1990. Interleukin 1 is an autocrine regulator of human endothelial cell growth. Proc Natl Acad Sci U S A. 87:6487-91.
Croner RS, Foertsch T, Brueckl WM, Guenther K, Siebenhaar R, Stremmel C, Matzel KE, Papadopoulos T, Kirchner T, Behrens J, Klein-Hitpass L, Stuerzl M, Hohenberger W and Reingruber B. Common denominator genes that distinguish colorectal carcinoma from normal mucosa. Int J Colorectal Dis 2005a; 20(4):353-62.
Croner RS, Guenther K, Foertsch T, Siebenhaar R, Brueckl WM, Stremmel C, Hlubek F, Hohenberger W and Reingruber B. Tissue preparation for gene expression profiling of colorectal carcinoma: three alternatives to laser microdissection with preamplification. J Lab Clin Med 2004; 143(6):344-51.
Croner RS, Peters A, Brueckl WM, Matzel KE, Klein-Hitpass L, Brabletz T, Papadopoulos T, Hohenberger W, Reingruber B and Lausen B. Microarray versus conventional prediction of lymph node metastasis in colorectal carcinoma. Cancer 2005b; 104(2):395-404.
Ehlert JE, Addison CA, Burdick MD, Kunkel SL and Strieter RM. Identification and partial characterization of a variant of human CXCR3 generated by posttranscriptional exon skipping. J Immunol 2004; 173(10):6234-40.
Fajardo, L.F., H.H. Kwan, J. Kowalski, S.D. Prionas, and A.C. Allison. 1992. Dual role of tumor necrosis factor-alpha in angiogenesis. Am J Pathol. 140:539-44.
Farrell RJ and Peppercorn MA. Ulcerative colitis. Lancet 2002; 359(9303):331-40.
Fathallah-Shaykh, H.M., L.J. Zhao, A.I. Kafrouni, G.M. Smith, and J. Forman. 2000. Gene transfer of IFN-gamma into established brain tumors represses growth by antiangiogenesis. Jlmmunol. 164:217-22.
Frater-Schroder, M., W. Risau, R. Hallmann, P. Gautschi, and P. Bohlen. 1987. Tumor necrosis factor type alpha, a potent inhibitor of endothelial cell growth in vitro, is angiogenic in vivo. Proc Natl Acad Sci U S A. 84:5277-81.
Friesel, R., A. Komoriya, and T. Maciag. 1987. Inhibition of endothelial cell proliferation by gamma-interferon. J Cell Biol. 104:689-96.
Gerol, M., L. Curry, L. McCarroll, S. Doctrow, and A. RayChaudhury. 1998. Growth regulation of cultured endothelial cells by inflammatory cytokines: mitogenic, anti-proliferative and cytotoxic effects. Comp Biochem Physiol C Pharmacol Toxicol Endocrinol. 120:397-404.
Guenzi, E., K. Töpolt, E. Cornali, C. Lubeseder-Martellato, A. Jörg, K. Matzen, C. Zietz, E. Kremmer, F. Nappi, M. Schwemmle, C. Hohenadl, G. Barillari, E. Tschachler, P. Monini, B. Ensoli, and M. Stürzl. 2001. The helical domain of GBP-1 mediates the inhibition of endothelial cell proliferation by inflammatory cytokines. Embo J. 20:5568-77.
Greenwood M. The Natural Duration of Cancer. Rep Publ Hlth Med Subj 1926; 33(London. HM Stationary Office.):
(Guenzi, E., K. Töpolt, C. Lubeseder-Martellato, A. Jörg, E. Naschberger, R. Benelli, A. Albini, and M. Stü rzl. 2003. The guanylate binding protein-1 GTPase controls the invasive and angiogenic capability of endothelial cells through inhibition of MMP-1 expression. Embo J. 22:3772-82.
Guenzi E, Töpolt K, Cornali E, Lubeseder-Martellato C, Jörg A, Matzen K, Zietz C, Kremmer E, Nappi F, Schwemmle M, Hohenadl C, Barillari G, Tschachler E, Monini P, Ensoli B and Stürzl M. The helical domain of GBP-1 mediates the inhibition of endothelial cell proliferation by inflammatory cytokines. Embo J 2001; 20(20):5568-77.
Guenzi E, Töpolt K, Lubeseder-Martellato C, Jörg A, Naschberger E, Benelli R, Albini A and Stürzl M. The guanylate binding protein-1 GTPase controls the invasive and angiogenic capability of endothelial cells through inhibition of MMP-1 expression. Embo J 2003; 22(15):3772-82.
Hawk, E.T., and B. Levin. 2005. Colorectal cancer prevention. J Clin Oncol. 23:378-91. Hurwitz, H., L. Fehrenbacher, W. Novotny, T. Cartwright, J. Hainsworth, W. Heim, J. Berlin, A. Baron, S. Griffing, E. Holmgren, N. Ferrara, G. Fyfe, B. Rogers, R. Ross, and F. Kabbinavar. 2004. Bevacizumab plus irinotecan, fluorouracil, and leucovorin for metastatic colorectal cancer. N Engl J Med. 350:2335-42.
Ilyas, M., J. Straub, I.P. Tomlinson, and W.F. Bodmer. 1999. Genetic pathways in colorectal and other cancers. Eur J Cancer. 35:1986-2002.
Ishigami, S.I., S. Arii, M. Furutani, M. Niwano, T. Harada, M. Mizumoto, A. Mori, H. Onodera, and M. Imamura. 1998. Predictive value of vascular endothelial growth factor (VEGF) in metastasis and prognosis of human colorectal cancer. Br J Cancer. 78:1379-84.
Itzkowitz SH and Yio X. Inflammation and cancer IV. Colorectal cancer in inflammatory bowel disease: the role of inflammation. Am J Physiol Gastrointest Liver Physiol 2004; 287(1):G7-17.
Jass JR and Sobin LH (1989). Histological Classification of Tumours. Berlin Heidelberg New York, Springer.
Jass, J.R. 2002. Pathogenesis of colorectal cancer. Surg Clin North Am. 82:891-904.
Joseph, I.B., and J.T. Isaacs. 1998. Macrophage role in the anti-prostate cancer response to one class of antiangiogenic agents. J Natl Cancer Inst. 90:1648-53.
Kahlenberg, M.S., J.M. Sullivan, D.D. Witmer, and N.J. Petrelli. 2003. Molecular prognostics in colorectal cancer. Surg Oncol. 12:173-86.
Kang, S.M., K. Maeda, N. Onoda, Y.S. Chung, B. Nakata, Y. Nishiguchi, and M. Sowa. 1997. Combined analysis of p53 and vascular endothelial growth factor expression in colorectal carcinoma for determination of tumor vascularity and liver metastasis. Int J Cancer. 74:502-7.
Kumar, H., K. Heer, P.W. Lee, G.S. Duthie, A.W. MacDonald, J. Greenman, M.J. Kerin, and J.R. Monson. 1998. Preoperative serum vascular endothelial growth factor can predict stage in colorectal cancer. Clin Cancer Res. 4:1279-85.
Lasagni L, Francalanci M, Annunziato F, Lazzeri E, Giannini S, Cosmi L, Sagrinati C, Mazzinghi B, Orlando C, Maggi E, Marra F, Romagnani S, Serio M and Romagnani P. An alternatively spliced variant of CXCR3 mediates the inhibition of endothelial cell growth induced by IP-10, Mig, and I-TAC, and acts as functional receptor for platelet factor 4. J Exp Med 2003; 197(11):137-49.
Lubeseder-Martellato C, Guenzi E, Jörg A, Töpolt K, Naschberger E, Kremmer E, Zietz C, Tschachler E, Hutzler P, Schwemmle M, Matzen K, Grimm T, Ensoli B and Stürzl M. Guanylate-Binding Protein-1 Expression Is Selectively Induced by Inflammatory Cytokines and Is an Activation Marker of Endothelial Cells during Inflammatory Diseases. Am J Pathol 2002; 161(5):1749-59.
Mahadevan, V., I.R. Hart, and G.P. Lewis. 1989. Factors influencing blood supply in wound granuloma quantitated by a new in vivo technique. Cancer Res. 49:415-9.
Montrucchio, G., E. Lupia, E. Battaglia, G. Passerini, F. Bussolino, G. Emanuelli, and G. Camussi. 1994. Tumor necrosis factor alpha-induced angiogenesis depends on in situ platelet-activating factor biosynthesis. J Exp Med. 180:377-82.
Naschberger E, Bauer M and Stürzl M. Human guanylate binding protein-1 (hGBP-1) characterizes and establishes a non-angiogenic endothelial cell activation phenotype in inflammatory diseases. Adv Enzyme Regul 2005; 45(215-27.
Naschberger E, Werner T, Vicente AB, Guenzi E, Töpolt K, Leubert R, Lubeseder-Martellato C, Nelson PJ and Stürzl M. Nuclear factor-kappaB motif and interferon-alpha-stimulated response element cooperate in the activation of guanylate-binding protein-1 expression by inflammatory cytokines in endothelial cells. Biochem J 2004; 379(Pt 2):409-20.
Naschberger, E., M. Bauer, and M. Sturzl. 2005. Human guanylate binding protein-1 (hGBP-1) characterizes and establishes a non-angiogenic endothelial cell activation phenotype in inflammatory diseases. Adv Enzyme Regul*.*
Negrier S, Escudier B, Lasset C, Douillard JY, Savary J, Chevreau C, Ravaud A, Mercatello A, Peny J, Mousseau M, Philip T and Tursz T. Recombinant human interleukin-2, recombinant human interferon alfa-2a, or both in metastatic renal-cell carcinoma. Groupe Francais d'Immunotherapie. N Engl J Med 1998; 338(18):1272-8.
Norioka, K., T. Mitaka, Y. Mochizuki, M. Hara, M. Kawagoe, and H. Nakamura. 1994. Interaction of interleukin-1 and interferon-gamma on fibroblast growth factor-induced angiogenesis. Jpn J Cancer Res. 85:522-9.
Prakash B, Praefcke GJ, Renault L, Wittinghofer A and Herrmann C. Structure of human guanylate-binding protein I representing a unique class of GTP-binding proteins. Nature 2000; 403(6769):567-71.
Romagnani P, Annunziato F, Lasagni L, Lazzeri E, Beltrame C, Francalanci M, Uguccioni M, Galli G, Cosmi L, Maurenzig L, Baggiolini M, Maggi E, Romagnani S and Serio M. Cell cycle-dependent expression of CXC chemokine receptor 3 by endothelial cells mediates angiostatic activity. J Clin Invest 2001; 107(1):53-63.
Romagnani P, Lasagni L, Annunziato F, Serio M and Romagnani S. CXC chemokines: the regulatory link between inflammation and angiogenesis. Trends Immunol 2004; 25(4):201-9.
Samaniego, F., P.D. Markham, R. Gendelman, R.C. Gallo, and B. Ensoli. 1997. Inflammatory cytokines induce endothelial cells to produce and release basic fibroblast growth factor and to promote Kaposi's sarcoma-like lesions in nude mice. J Immunol. 158:1887-94.
Schweigerer, L., B. Malerstein, and D. Gospodarowicz. 1987. Tumor necrosis factor inhibits the proliferation of cultured capillary endothelial cells. Biochem Biophys Res Commun. 143:997-1004.
Smith, R.A., V. Cokkinides, A.C. von Eschenbach, B. Levin, C. Cohen, C.D. Runowicz, S. Sener, D. Saslow, and H.J. Eyre. 2002. American Cancer Society guidelines for the early detection of cancer. CA Cancer J Clin. 52:8-22.
Soreide O, Norstein J, Fielding LP and Silen W (1997). International standardization and documentation of the treatment of rectal cancer. Berlin Heidelberg New York, Springer.
Spinetti G, Camarda G, Bemardini G, Romano Di Peppe S, Capogrossi MC and Napolitano M. The chemokine CXCL13 (BCA-1) inhibits FGF-2 effects on endothelial cells. Biochem Biophys Res Commun 2001; 289(1):19-24.
Strieter RM, Belperio JA, Burdick MD and Keane MP. CXC chemokines in angiogenesis relevant to chronic fibroproliferation. Curr Drug Targets Inflamm Allergy 2005a; 4(1):23-6.
Strieter RM, Belperio JA, Phillips RJ and Keane MP. CXC chemokines in angiogenesis of cancer. Semin Cancer Biol 2004; 14(3):195-200.
Strieter RM, Burdick MD, Gomperts BN, Belperio JA and Keane MP. CXC chemokines in angiogenesis. Cytokine Growth Factor Rev 2005b; 16(6):593-609.
Strieter RM, Burdick MD, Mestas J, Gomperts B, Keane MP and Belperio JA. Cancer CXC chemokine networks and tumour angiogenesis. Eur J Cancer 2006; 42(6):768-778.
Stürzl M, Hohenadl C, Zietz C, Castanos-Velez E, Wunderlich A, Ascherl G, Biberfeld P, Monini P, Browning PJ and Ensoli B. Expression of K13/v-FLIP gene of human herpesvirus 8 and apoptosis in Kaposi's sarcoma spindle cells. J Natl Cancer Inst 1999; 91(20):1725-33.
Sturzl M, Roth WK, Brockmeyer NH, Zietz C, Speiser B and Hofschneider PH. Expression of platelet-derived growth factor and its receptor in AIDS-related Kaposi sarcoma in vivo suggests paracrine and autocrine mechanisms of tumor maintenance. Proc Natl Acad Sci U S A 1992; 89(15):7046-50.
Takayama, T., M. Ohi, T. Hayashi, K. Miyanishi, A. Nobuoka, T. Nakajima, T. Satoh, R. Takimoto, J. Kato, S. Sakamaki, and Y. Niitsu. 2001. Analysis of K-ras, APC, and beta-catenin in aberrant crypt foci in sporadic adenoma, cancer, and familial adenomatous polyposis. Gastroenterology. 121:599-611.
Takebayashi, Y., S. Aklyama, K. Yamada, S. Akiba, and T. Aikou. 1996. Angiogenesis as an unfavorable prognostic factor in human colorectal carcinoma. Cancer. 78:226-31.
Torisu, H., M. Ono, H. Kiryu, M. Furue, Y. Ohmoto, J. Nakayama, Y. Nishioka, S. Sone, and M. Kuwano. 2000. Macrophage infiltration correlates with tumor stage and angiogenesis in human malignant melanoma: possible involvement of TNFalpha and IL-lalpha. Int J Cancer. 85:182-8.
Vogelstein, B., E.R. Fearon, S.R. Hamilton, S.E. Kern, A.C. Preisinger, M. Leppert, Y. Nakamura, R. White, A.M. Smits, and J.L. Bos. 1988. Genetic alterations during colorectal-tumor development. N Engl J Med. 319:525-32.
Yilmaz, A., G. Bieler, O. Spertini, F.J. Lejeune, and C. Ruegg. 1998. Pulse treatment of human vascular endothelial cells with high doses of tumor necrosis factor and interferon-gamma results in simultaneous synergistic and reversible effects on proliferation and morphology. Int J Cancer. 77:592-9.

## Claims

1. An *ex vivo* method for the detection of an angiostatic tumor stage/tumor area of colorectal carcinoma (CRC) in a patient comprising a detection step using a microarray, wherein the microarray comprises gene probes specifically hybridizing to the nucleic acid sequences of SEQ ID NO: 5, 1, 3, and 7, wherein the detection of said nucleic acid sequences is indicative for the presence of an angiostatic tumor stage/tumor area of CRC in said patient.

2. The method of claim 1, wherein the array additionally contains appropriate control gene probes, e.g. actin or GAPDH.

3. The method of claims 1 or 2, wherein the array in addition at least comprises gene probes capable of hybridizing to the nucleic acid sequences of SEQ ID NO: 9, 11, 12 and 14.

4. The method of claims 1-3 wherein the array additionally contains gene probes capable of specifically hybridizing to nucleic acids encoding VEGF, bFGF and/or wherein the gene probes are oligonucleotides, cDNA, RNA or PNA molecules.

5. The method of claim 1, wherein the nucleic acids are labelled, wherein the label preferably is selected from a radioactive, fluorescence, biotin, digoxigenin, peroxidase labelling or a labelling detectable by alkaline phosphatase.

6. The method of claim 1, wherein the gene probes of the array are bound to a solid phase matrix, e.g. a nylon membrane, glass or plastics.

7. An *ex vivo* method for the diagnosis of an angiostatic tumor stage/tumor area in a CRC patient comprising the steps of:
a) providing a sample of the patient;
b) extracting RNA from the sample;
c) optionally transcribing RNA to cDNA or cRNA;
d) detecting, whether the sample contains at least the nucleic acid sequences of SEQ ID NO: 5, 1, 3, and 7;
e) wherein the presence of said nucleic acids is indicative for the presence of an angiostatic tumor stage/tumor area of CRC in said patient.

8. The method of claim 7, wherein the sample is a CRC tissue sample or a cell lysate or a body fluid sample, wherein the detection preferably is performed by RT-PCR, and wherein the RT-PCR preferably is multiplex RT-PCR.

9. The method of claim 7, wherein the detection is performed by means of complementary gene probes, wherein the gene probes preferably are cDNA or oligonucleotide probes, and wherein the detection preferably is performed by means of gene probes, which are capable of hybridizing to at least a portion of the nucleic acid sequences of SEQ ID NO: 5, 1, 3, 7, 9, 11, 12 and 14.

10. The method of claim 9, wherein a microarray as defined in claims 1-7 is used for the detection, or wherein the hybridization is performed under moderatly stringent conditions.

11. An *ex vivo* method for the diagnosis of an angiostatic tumor stage/tumor area in a CRC patient comprising the steps of:
a) providing a sample from the patient;
b) detecting, whether the sample contains at least the amino acids corresponding to the nucleic acid sequences of SEQ ID NO: 5, 1, 3, and 7;
c) wherein the presence of said amino acids is indicative for the presence of an angiostatic tumor stage/tumor area of CRC in said patient.

12. The method of claim 11, wherein the detection is performed by contacting the sample with antibodies, which specifically recognize an amino acid expressed from a nucleic acid sequence of one of SEQ ID NO: 5, 1, 3, 7, 9, 11, 12 and 14, or wherein the sample is a CRC tissue sample, a cell lysat or a body fluid, or wherein the amino acid sequences are detected by means of multiplex Western blot or ELISA.

13. An *ex vivo* method for the prediction of responses to therapy of CRC patients and patients with other diseases comprising the steps of:
a) providing a sample of the patient;
b) extracting RNA from the sample;
c) optionally transcribing RNA to cDNA or cRNA;
d) detecting, whether the sample contains at least the nucleic acid sequences of SEQ ID NO: 5, 1, 3, and 7;
e) wherein the presence of said nucleic acids is indicative for the presence of a specific therapy response or non-response of said patients.

## Patentansprüche

1. *Ex vivo*-Verfahren zum Nachweis einer angiostatischen Tumorstufe bzw. eines angiostatischen Tumorbereichs von Dickdarmkrebs (Colorectal carcinoma, CRC) bei einem Patienten, wobei das Verfahren einen Schritt des Nachweises unter Verwendung eines Mikroarrays umfasst, wobei das Mikroarray Gensonden umfasst, die spezifisch zu den Nukleinsäuresequenzen von SEQ ID NO: 5, 1, 3 und 7 hybridisieren, wobei der Nachweis der Nukleinsäuresequenzen das Vorhandensein einer angiostatischen Tumorstufe bzw. eines angiostatischen Tumorbereichs von CRC in dem Patienten anzeigt.

2. Verfahren nach Anspruch 1, wobei das Array außerdem zweckmäßige Kontrollgensonden umfasst, z. B. Actin oder GAPDH.

3. Verfahren nach den Ansprüchen 1 oder 2, wobei das Array zusätzlich mindestens Gensonden umfasst, die in der Lage sind, zu den Nukleinsäuresequenzen von SEQ ID NO: 9, 11, 12 und 14 zu hybridisieren.

4. Verfahren nach den Ansprüchen 1-3, wobei das Array außerdem Gensonden umfasst, die in der Lage sind, spezifisch zu Nukleinsäuren zu hybridisieren, die VEGF und bFGF codieren, und/oder wobei die Gensonden Oligonukleotide, cDNA-, RNA- oder PNA-Moleküle sind.

5. Verfahren nach Anspruch 1, wobei die Nukleinsäuren markiert sind, wobei die Markierung bevorzugt aus einer Radioaktiv-, Fluoreszenz-, Biotin-, Digoxigenin- oder Peroxidase-Markierung oder einer Markierung, die durch alkalische Phosphatase nachgewiesen werden kann, ausgewählt wird.

6. Verfahren nach Anspruch 1, wobei die Gensonden des Arrays an eine Festphasenmatrix gebunden sind, z. B. eine Nylonmembran, Glas oder Kunststoff.

7. *Ex vivo*-Verfahren für die Diagnose einer angiostatischen Tumorstufe bzw. eines angiostatischen Tumorbereichs bei einem CRC-Patienten, wobei das Verfahren folgende Schritte umfasst:
a) Bereitstellen einer Probe des Patienten;
b) Extrahieren von RNA aus der Probe;
c) optionales Transkribieren von RNA zu cDNA oder cRNA;
d) Nachweisen, ob die Probe mindestens die Nukleinsäuresequenzen von SEQ ID NO: 5, 1, 3 und 7 enthält;
e) wobei das Vorhandensein der Nukleinsäuren das Vorhandensein einer angiostatischen Tumorstufe bzw. eines angiostatischen Tumorbereichs von CRC in dem Patienten anzeigt.

8. Verfahren nach Anspruch 7, wobei die Probe eine CRC-Gewebeprobe oder ein Zelllysat oder eine Körperfluidprobe ist, wobei der Nachweis bevorzugt durch eine RT-PCR ausgeführt wird, und wobei die RT-PCR bevorzugt eine Multiplex-RT-PCR ist.

9. Verfahren nach Anspruch 7, wobei der Nachweis mittels komplementärer Gensonden ausgeführt wird, wobei die Gensonden bevorzugt cDNA- oder Oligonukleotid-Sonden sind und wobei der Nachweis bevorzugt mittels Gensonden ausgeführt wird, die in der Lage sind, zu mindestens einem Abschnitt der Nukleinsäuresequenzen von SEQ ID NO: 5, 1, 3, 7, 9, 11, 12 und 14 zu hybridisieren.

10. Verfahren nach Anspruch 9, wobei ein Mikroarray nach den Ansprüchen 1-7 zum Nachweisen verwendet wird oder wobei die Hybridisierung unter moderat stringenten Bedingungen ausgeführt wird.

11. Ex vivo-Verfahren für die Diagnose einer angiostatischen Tumorstufe bzw. eines angiostatischen Tumorbereichs bei einem CRC-Patienten, wobei das Verfahren folgende Schritte umfasst:
a) Bereitstellen einer Probe von dem Patienten;
b) Nachweisen, ob die Probe mindestens die Aminosäuren enthält, die den Nukleinsäuresequenzen von SEQ ID NO: 5, 1, 3 und 7 entsprechen;
c) wobei das Vorhandensein der Aminosäuren das Vorhandensein einer angiostatischen Tumorstufe bzw. eines angiostatischen Tumorbereichs von CRC in dem Patienten anzeigt.

12. Verfahren nach Anspruch 11, wobei der Nachweis erbracht wird, indem die Probe mit Antikörpern in Kontakt gebracht wird, die spezifisch eine Aminosäure erkennen, die aus einer Nukleinsäuresequenz eines von SEQ ID NO: 5, 1, 3, 7, 9, 11, 12 und 14 exprimiert wird, oder wobei die Probe eine CRC-Gewebeprobe, ein Zelllysat oder ein Körperfluid ist oder wobei die Aminosäuresequenzen mittels eines Multiplex-Western-Blot oder ELISA nachgewiesen werden.

13. Ex vivo-Verfahren zum Vorhersagen des Ansprechens von CRC-Patienten und Patienten mit anderen Krankheiten auf eine Therapie, wobei das Verfahren folgende Schritte umfasst:
a) Bereitstellen einer Probe des Patienten;
b) Extrahieren von RNA aus der Probe;
c) optionales Transkribieren von RNA zu cDNA oder cRNA;
d) Nachweisen, ob die Probe mindestens die Nukleinsäuresequenzen von SEQ ID NO: 5, 1, 3 und 7 enthält;
e) wobei das Vorhandensein der Nukleinsäuren das Vorhandensein eines spezifischen Ansprechens oder Nicht-Ansprechens der Patienten anzeigt.

## Revendications

1. Procédé *ex vivo* pour la détection d'un stade de tumeur/zone de tumeur angiostatique du carcinome colorectal (CRC) chez un patient comprenant une étape de détection utilisant un microréseau, dans lequel le microréseau comprend des sondes génétiques s'hybridant spécifiquement aux séquences d'acide nucléique de SEQ ID n° 5, 1, 3 et 7, dans lequel la détection desdites séquences d'acide nucléique est indicatrice de la présence d'un stade de tumeur/zone de tumeur angiostatique de CRC chez ledit patient.

2. Procédé selon la revendication 1, dans lequel le réseau contient de plus des sondes génétiques de contrôle appropriées, par exemple de l'actine ou de la GAPDH.

3. Procédé selon les revendications 1 ou 2, dans lequel le réseau comprend de plus au moins les sondes génétiques capables de s'hybrider aux séquences d'acide nucléique de SEQ ID 9, 11, 12 et 14.

4. Procédé selon les revendications 1 à 3 dans lequel le réseau contient de plus des sondes génétiques capables de s'hybrider spécifiquement aux acides nucléiques codant VEGF, bFGF et/ou dans lequel les sondes génétiques sont des oligonucléotides, des molécules d'ADNc, d'ARN ou de PNA.

5. Procédé selon la revendication 1, dans lequel les acides nucléiques sont marqués, dans lequel le marqueur est de préférence choisi parmi un marqueur radioactif, de fluorescence, biotine, digoxigénine, peroxydase ou un marqueur détectable par alcaline phosphatase.

6. Procédé selon la revendication 1, dans lequel les sondes génétiques du réseau sont liées à une matrice en phase solide, par exemple une membrane de nylon, du verre ou du plastique.

7. Procédé *ex vivo* pour le diagnostic d'un stade de tumeur/zone de tumeur angiostatique chez un patient CRC comprenant les étapes de :
a) fournir un échantillon du patient ;
b) extraire l'ARN de l'échantillon ;
c) éventuellement transcrire l'ARN en ADNc ou en ARNc ;
d) détecter si l'échantillon contient au moins les séquences d'acide nucléique de SEQ ID n° 5, 1, 3 et 7 ;
e) dans lequel la présence desdits acides nucléiques est indicatrice de la présence d'un stade de tumeur/zone de tumeur angiostatique de CRC chez ledit patient.

8. Procédé selon la revendication 7, dans lequel l'échantillon est un échantillon de tissu de CRC ou un lysat cellulaire ou un échantillon de fluide corporel, dans lequel la détection est réalisée de préférence par RT-PCR, et dans lequel la RT-PCR est de préférence une RT-PCR multiplexe.

9. Procédé selon la revendication 7, dans lequel la détection est réalisée au moyen de sondes génétiques complémentaires, dans lequel les sondes génétiques sont de préférence des ADNc ou des sondes oligonucléotidiques, et dans lequel la détection est réalisée de préférence au moyen de sondes génétiques, qui sont capables de s'hybrider à au moins une partie des séquences d'acide nucléique de SEQ ID n° 5, 1, 3, 7, 9, 11, 12 et 14.

10. Procédé selon la revendication 9, dans lequel un microréseau tel que défini dans les revendications 1 à 7 est utilisé pour la détection, ou dans lequel l'hybridation est réalisée dans des conditions modérément stringentes.

11. Procédé *ex vivo* pour le diagnostic d'un stade de tumeur/zone de tumeur angiostatique chez un patient CRC comprenant les étapes de :
a) fournir un échantillon du patient ;
b) détecter si l'échantillon contient au moins les acides aminés correspondant aux séquences d'acide nucléique de SEQ ID n° 5, 1, 3 et 7 ;
c) dans lequel la présence desdits acides aminés est indicatrice de la présence d'un stade de tumeur/zone de tumeur angiostatique de CRC chez ledit patient.

12. Procédé selon la revendication 11, dans lequel la détection est réalisée par la mise en contact de l'échantillon avec des anticorps, qui reconnaissent spécifiquement un acide aminé exprimé à partir d'une séquence d'acide nucléique de l'une parmi SEQ ID n° 5, 1, 3, 7, 9, 11, 12 et 14, ou dans lequel l'échantillon est un échantillon de tissu CRC, un lysat cellulaire ou un fluide corporel, ou dans lequel les séquences d'acides aminés sont détectées au moyen d'un blot Western multiplexe ou d'un ELISA.

13. Procédé *ex vivo* pour la prédiction de réponses à une thérapie de patients CRC et de patients avec d'autres maladies comprenant les étapes de :
a) fournir un échantillon du patient ;
b) extraire l'ARN de l'échantillon ;
c) éventuellement transcrire l'ARN en ADNc ou en ARNc ;
d) détecter si l'échantillon contient au moins les séquences d'acide nucléique de SEQ ID n° 5, 1, 3 et 7 ;
e) dans lequel la présence desdits acides nucléiques est indicatrice de la présence d'une réponse de thérapie spécifique ou de non réponse desdits patients.


### SEQUENCE LISTING

<110> Friedrich- Alexander - Universität Erlangen- Nür nber g
<120> Mcroarray for the detection of an angiostatic tumor stage of colorectal carcinoma
<130> P23867
<150> US 60/861, 624
   <151> 2006-11-29
<160> 15
<170> Patent Inversion 3.3
<210> 1
   <211> 2545
   <212> DNA
   <213> Homo sapiens
<400> 1
   atccaataca ggagtgactt ggaactccat tctatcacta tgaagaaaag tggtgttctt 60
   ttcctcttgg gcatcatctt gctggttctg attggagtgc aaggaacccc agtagtgaga 120
   aagggtcgct gttcctgcat cagcaccaac caagggacta tccacctaca atccttgaaa 180
   gaccttaaac aatttgcccc aagcccttcc tgcgagaaaa ttgaaatcat tgctacactg 240
   aagaatggag ttcaaacatg tctaaaccca gattcagcag atgtgaagga actgattaaa 300
   aagtgggaga aacaggtcag ccaaaagaaa aagcaaaaga atgggaaaaa acatcaaaaa 360
   aagaaagttc tgaaagttcg aaaatctcaa cgttctcgtc aaaagaagac tacataagag 420
   accacttcac caataagtat tctgtgttaa aaatgttcta ttttaattat accgctatca 480
   ttccaaagga ggatggcata taatacaaag gcttattaat ttgactagaa aatttaaaac 540
   attactctga aattgtaact aaagttagaa agttgatttt aagaatccaa acgttaagaa 600
   ttgttaaagg ctatgattgt ctttgttctt ctaccaccca ccagttgaat ttcatcatgc 660
   ttaaggccat gattttagca atacccatgt ctacacagat gttcacccaa ccacatccca 720
   ctcacaacag ctgcctggaa gagcagccct aggcttccac gtactgcagc ctccagagag 780
   tatctgaggc acatgtcagc aagtcctaag cctgttagca tgctggtgag ccaagcagtt 840
   tgaaattgag ctggacctca ccaagctgct gtggccatca acctctgtat ttgaatcagc 900
   ctacaggcct cacacacaat gtgtctgaga gattcatgct gattgttatt gggtatcacc 960
   actggagatc accagtgtgt ggctttcaga gcctcctttc tggctttgga agccatgtga 1020
   ttccatcttg cccgctcagg ctgaccactt tatttctttt tgttcccctt tgcttcattc 1080
   aagtcagctc ttctccatcc taccacaatg cagtgccttt cttctctcca gtgcacctgt 1140
   catatgctct gatttatctg agtcaactcc tttctcatct tgtccccaac accccacaga 1200
   agtgctttct tctcccaatt catcctcact cagtccagct tagttcaagt cctgcctctt 1260
   aaataaacct ttttggacac acaaattatc ttaaaactcc tgtttcactt ggttcagtac 1320
   cacatgggtg aacactcaat ggttaactaa ttcttgggtg tttatcctat ctctccaacc 1380
   agattgtcag ctccttgagg gcaagagcca cagtatattt ccctgtttct tccacagtgc 1440
   ctaataatac tgtggaacta ggttttaata attttttaat tgatgttgtt atgggcagga 1500
   tggcaaccag accattgtct cagagcaggt gctggctctt tcctggctac tccatgttgg 1560
   ctagcctctg gtaacctctt acttattatc ttcaggacac tcactacagg gaccagggat 1620
   gatgcaacat ccttgtcttt ttatgacagg atgtttgctc agcttctcca acaataagaa 1680
   gcacgtggta aaacacttgc ggatattctg gactgttttt aaaaaatata cagtttaccg 1740
   aaaatcatat aatcttacaa tgaaaaggac tttatagatc agccagtgac caaccttttc 1800
   ccaaccatac aaaaattcct tttcccgaag gaaaagggct ttctcaataa gcctcagctt 1860
   tctaagatct aacaagatag ccaccgagat ccttatcgaa actcatttta ggcaaatatg 1920
   agttttattg tccgtttact tgtttcagag tttgtattgt gattatcaat taccacacca 1980
   tctcccatga agaaagggaa cggtgaagta ctaagcgcta gaggaagcag ccaagtcggt 2040
   tagtggaagc atgattggtg cccagttagc ctctgcagga tgtggaaacc tccttccagg 2100
   ggaggttcag tgaattgtgt aggagaggtt gtctgtggcc agaatttaaa cctatactca 2160
   ctttcccaaa ttgaatcact gctcacactg ctgatgattt agagtgctgt ccggtggaga 2220
   tcccacccga acgtcttatc taatcatgaa actccctagt tccttcatgt aacttccctg 2280
   aaaaatctaa gtgtttcata aatttgagag tctgtgaccc acttaccttg catctcacag 2340
   gtagacagta tataactaac aaccaaagac tacatattgt cactgacaca cacgttataa 2400
   tcatttatca tatatataca tacatgcata cactctcaaa gcaaataatt tttcacttca 2460
   aaacagtatt gacttgtata ccttgtaatt tgaaatattt tctttgttaa aatagaatgg 2520
   tatcaataaa tagaccatta atcag 2545
<210> 2
   <211> 125
   <212> PRT
   <213> Homo sapiens
<400> 2
   Met Lys Lys Ser Gly Val Leu Phe Leu Leu Gly Ile Ile Leu Leu Val
   1 5 10 15
   Leu Ile Gly Val Gln Gly Thr Pro Val Val Arg Lys Gly Arg Cys Ser
   20 25 30
   Cys Ile Ser Thr Asn Gln Gly Thr Ile His Leu Gln Ser Leu Lys Asp
   35 40 45
   Leu Lys Gln Phe Ala Pro Ser Pro Ser Cys Glu Lys Ile Glu Ile Ile
   50 55 60
   Ala Thr Leu Lys Asn Gly Val Gln Thr Cys Leu Asn Pro Asp Ser Ala
   65 70 75 80
   Asp Val Lys Glu Leu Ile Lys Lys Trp Glu Lys Gln Val Ser Gln Lys
   85 90 95
   Lys Lys Gln Lys Asn Gly Lys Lys His Gln Lys Lys Lys Val Leu Lys
               100                 105                 110
   Val Arg Lys Ser Gln Arg Ser Arg Gln Lys Lys Thr Thr
           115                 120                 125
<210> 3
   <211> 1172
   <212> DNA
   <213> Homo sapiens
<400> 3
   gagacattcc tcaattgctt agacatattc tgagcctaca gcagaggaac ctccagtctc      60
   agcaccatga atcaaactgc gattctgatt tgctgcctta tctttctgac tctaagtggc     120
   attcaaggag tacctctctc tagaaccgta cgctgtacct gcatcagcat tagtaatcaa     180
   cctgttaatc caaggtcttt agaaaaactt gaaattattc ctgcaagcca attttgtcca     240
   cgtgttgaga tcattgctac aatgaaaaag aagggtgaga agagatgtct gaatccagaa     300
   tcgaaggcca tcaagaattt actgaaagca gttagcaagg aaatgtctaa aagatctcct     360
   taaaaccaga ggggagcaaa atcgatgcag tgcttccaag gatggaccac acagaggctg     420
   cctctcccat cacttcccta catggagtat atgtcaagcc ataattgttc ttagtttgca     480
   gttacactaa aaggtgacca atgatggtca ccaaatcagc tgctactact cctgtaggaa     540
   ggttaatgtt catcatccta agctattcag taataactct accctggcac tataatgtaa     600
   gctctactga ggtgctatgt tcttagtgga tgttctgacc ctgcttcaaa tatttccctc     660
   acctttccca tcttccaagg gtactaagga atctttctgc tttggggttt atcagaattc     720
   tcagaatctc aaataactaa aaggtatgca atcaaatctg ctttttaaag aatgctcttt     780
   acttcatgga cttccactgc catcctccca aggggcccaa attctttcag tggctaccta     840
   catacaattc caaacacata caggaaggta gaaatatctg aaaatgtatg tgtaagtatt     900
   cttatttaat gaaagactgt acaaagtata agtcttagat gtatatattt cctatattgt     960
   tttcagtgta catggaataa catgtaatta agtactatgt atcaatgagt aacaggaaaa    1020
   ttttaaaaat acagatagat atatgctctg catgttacat aagataaatg tgctgaatgg    1080
   ttttcaaata aaaatgaggt actctcctgg aaatattaag aaagactatc taaatgttga    1140
   aagatcaaaa ggttaataaa gtaattataa ct                                  1172
<210> 4
   <211> 98
   <212> PRT
   <213> Homo sapiens
<400> 4
   Met Asn Gln Thr Ala Ile Leu Ile Cys Cys Leu Ile Phe Leu Thr Leu
   1 5 10 15
   Ser Gly Ile Gln Gly Val Pro Leu Ser Arg Thr Val Arg Cys Thr Cys
   20 25 30
   Ile Ser Ile Ser Asn Gln Pro Val Asn Pro Arg Ser Leu Glu Lys Leu
   35 40 45
   Glu Ile Ile Pro Ala Ser Gln Phe Cys Pro Arg Val Glu Ile Ile Ala
   50 55 60
   Thr Met Lys Lys Lys Gly Glu Lys Arg Cys Leu Asn Pro Glu Ser Lys
   65 70 75 80
   Ala Ile Lys Asn Leu Leu Lys Ala Val Ser Lys Glu Met Ser Lys Arg
   85 90 95
   Ser Pro
<210> 5
   <211> 1493
   <212> DNA
   <213> Homo sapiens
<400> 5
   ttcctttcat gttcagcatt tctactcctt ccaagaagag cagcaaagct gaagtagcag 60
   caacagcacc agcagcaaca gcaaaaaaca aacatgagtg tgaagggcat ggctatagcc 120
   ttggctgtga tattgtgtgc tacagttgtt caaggcttcc ccatgttcaa aagaggacgc 180
   tgtctttgca taggccctgg ggtaaaagca gtgaaagtgg cagatattga gaaagcctcc 240
   ataatgtacc caagtaacaa ctgtgacaaa atagaagtga ttattaccct gaaagaaaat 300
   aaaggacaac gatgcctaaa tcccaaatcg aagcaagcaa ggcttataat caaaaaagtt 360
   gaaagaaaga atttttaaaa atatcaaaac atatgaagtc ctggaaaagg gcatctgaaa 420
   aacctagaac aagtttaact gtgactactg aaatgacaag aattctacag taggaaactg 480
   agacttttct atggttttgt gactttcaac ttttgtacag ttatgtgaag gatgaaaggt 540
   gggtgaaagg accaaaaaca gaaatacagt cttcctgaat gaatgacaat cagaattcca 600
   ctgcccaaag gagtccagca attaaatgga tttctaggaa aagctacctt aagaaaggct 660
   ggttaccatc ggagtttaca aagtgctttc acgttcttac ttgttgtatt atacattcat 720
   gcatttctag gctagagaac cttctagatt tgatgcttac aactattctg ttgtgactat 780
   gagaacattt ctgtctctag aagttatctg tctgtattga tctttatgct atattactat 840
   ctgtggttac agtggagaca ttgacattat tactggagtc aagcccttat aagtcaaaag 900
   catctatgtg tcgtaaagca ttcctcaaac attttttcat gcaaatacac acttctttcc 960
   ccaaatatca tgtagcacat caatatgtag ggaaacattc ttatgcatca tttggtttgt 1020
   tttataacca attcattaaa tgtaattcat aaaatgtact atgaaaaaaa ttatacgcta 1080
   tgggatactg gcaacagtgc acatatttca taaccaaatt agcagcaccg gtcttaattt 1140
   gatgtttttc aacttttatt cattgagatg ttttgaagca attaggatat gtgtgtttac 1200
   tgtacttttt gttttgatcc gtttgtataa atgatagcaa tatcttggac acatttgaaa 1260
   tacaaaatgt ttttgtctac caaagaaaaa tgttgaaaaa taagcaaatg tatacctagc 1320
   aatcactttt actttttgta attctgtctc ttagaaaaat acataatcta atcaatttct 1380
   ttgttcatgc ctatatactg taaaatttag gtatactcaa gactagttta aagaatcaaa 1440
   gtcatttttt tctctaataa actaccacaa cctttctttt ttaaaaaaaa aaa 1493
<210> 6
   <211> 94
   <212> PRT
   <213> Homo sapiens
<400> 6
   Met Ser Val Lys Gly Met Ala Ile Ala Leu Ala Val Ile Leu Cys Ala
   1 5 10 15
   Thr Val Val Gln Gly Phe Pro Met Phe Lys Arg Gly Arg Cys Leu Cys
   20 25 30
   Ile Gly Pro Gly Val Lys Ala Val Lys Val Ala Asp Ile Glu Lys Ala
   35 40 45
   Ser Ile Met Tyr Pro Ser Asn Asn Cys Asp Lys Ile Glu Val Ile Ile
   50 55 60
   Thr Leu Lys Glu Asn Lys Gly Gln Arg Cys Leu Asn Pro Lys Ser Lys
   65 70 75 80
   Gln Ala Arg Leu Ile Ile Lys Lys Val Glu Arg Lys Asn Phe
   85 90
<210> 7
   <211> 1803
   <212> DNA
   <213> Homo sapiens
<400> 7
   ggacatggca tcagagatcc acatgacagg cccaatgtgc ctcattgaga acactaatgg 60
   gcgactgatg gcgaatccag aagctctgaa gatcctttct gccattacac agcctatggt 120
   ggtggtggca attgtgggcc tctaccgcac aggcaaatcc tacctgatga acaagctggc 180
   tggaaagaaa aagggcttct ctctgggctc cacggtgcag tctcacacta aaggaatctg 240
   gatgtggtgt gtgccccacc ccaagaagcc aggccacatc ctagttctgc tggacaccga 300
   gggtctggga gatgtagaga agggtgacaa ccagaatgac tcctggatct tcgccctggc 360
   cgtcctcctg agcagcacct tcgtgtacaa tagcatagga accatcaacc agcaggctat 420
   ggaccaactg tactatgtga cagagctgac acatagaatc cgatcaaaat cctcacctga 480
   tgagaatgag aatgaggttg aggattcagc tgactttgtg agcttcttcc cagactttgt 540
   gtggacactg agagatttct ccctggactt ggaagcagat ggacaacccc tcacaccaga 600
   tgagtacctg acatactccc tgaagctgaa gaaaggtacc agtcaaaaag atgaaacttt 660
   taacctgccc agactctgta tccggaaatt cttcccaaag aaaaaatgct ttgtctttga 720
   tcggcccgtt caccgcagga agcttgccca gctcgagaaa ctacaagatg aagagctgga 780
   ccccgaattt gtgcaacaag tagcagactt ctgttcctac atctttagta attccaaaac 840
   taaaactctt tcaggaggca tccaggtcaa cgggcctcgt ctagagagcc tggtgctgac 900
   ctacgtcaat gccatcagca gtggggatct gccgtgcatg gagaacgcag tcctggcctt 960
   ggcccagata gagaactcag ctgcagtgca aaaggctatt gcccactatg aacagcagat 1020
   gggccagaag gtgcagctgc ccacagaaag cctccaggag ctgctggacc tgcacaggga 1080
   cagtgagaga gaggccattg aagtcttcat caggagttcc ttcaaagatg tggaccatct 1140
   atttcaaaag gagttagcgg cccagctaga aaaaaagcgg gatgactttt gtaaacagaa 1200
   tcaggaagca tcatcagatc gttgctcagc tttacttcag gtcattttca gtcctctaga 1260
   agaagaagtg aaggcgggaa tttattcgaa accagggggc tatcgtctct ttgttcagaa 1320
   gctacaagac ctgaagaaaa agtactatga ggaaccgagg aaggggatac aggctgaaga 1380
   gattctgcag acatacttga aatccaagga gtctatgact gatgcaattc tccagacaga 1440
   ccagactctc acagaaaaag aaaaggagat tgaagtggaa cgtgtgaaag ctgagtctgc 1500
   acaggcttca gcaaaaatgt tgcaggaaat gcaaagaaag aatgagcaga tgatggaaca 1560
   gaaggagagg agttatcagg aacacttgaa acaactgact gagaagatgg agaacgacag 1620
   ggtccagttg ctgaaagagc aagagaggac cctcgctctt aaacttcagg aacaggagca 1680
   actactaaaa gagggatttc aaaaagaaag cagaataatg aaaaatgaga tacaggatct 1740
   ccagacgaaa atgagacgac gaaaggcatg taccataagc taaagaccag agccttcctg 1800
   tca 1803
<210> 8
   <211> 592
   <212> PRT
   <213> Homo sapiens
<400> 8
<210> 9
   <211> 1776
   <212> DNA
   <213> Homo sapiens
<400> 9
   atggctccag agatcaactt gccgggccca atgagcctca ttgataacac taaagggcag    60
   ctggtggtga atccagaagc tctgaagatc ctatctgcaa ttacgcagcc tgtggtggtg   120
   gt ggcgat t g t gggcct ct a t cgcacaggc aaat cct acc t gat gaacaa gct ggct ggg 180
   aagaaaaacg gcttctctct aggctccaca gtgaagtctc acaccaaggg aatctggatg     240
   tggtgtgtgc ctcatcccaa gaagccagaa cacaccctag ttctgctcga cactgagggc     300
   ct gggagat a t agagaaggg t gacaat gag aat gact cct ggat ct t t gc ct t ggccat c 360
   ct cct gagca gcacct t cgt gt acaat agc at gggaacca t caaccagca ggccat ggac 420
   caact t cact at gt gacaga gct gacagat cgaat caagg caaact cct c acct ggt aac 480
   aat t ct gt ag acgact cagc t gact t t gt g agct t t t t t c cagcat t t gt gt ggact ct c 540
   agagatttca ccctggaact ggaagtagat ggagaaccca tcactgctga tgactacttg     600
   gagct t t cgc t aaagct aag aaaaggt act gat aagaaaa gt aaaagct t t aat gat cct 660
   cggt t gt gca t ccgaaagt t ct t ccccaag aggaagt gct t cgt ct t cga t t ggcccgct 720
   cct aagaagt acct t gct ca cct agagcag ct aaaggagg aagagct gaa ccct gat t t c 780
   at agaacaag t t gcagaat t t t gt t cct ac at cct cagcc at t ccaat gt caagact ct t 840
   t caggt ggca t t gcagt caa t gggcct cgt ct agagagcc t ggt gct gac ct acgt caat 900
   gccatcggca gtggggatct accctgcatg gagaacgcag tcctggcctt ggcccagata     960
   gagaactcag ccgcagtgga aaaggctatt gcccactatg aacagcagat gggccagaag    1020
   gtgcagctgc ccacggaaac cctccaggag ctgctggacc tgcacaggga cagtgagaga    1080
   gaggccat t g aagt ct t cat gaagaact ct t t caaggat g t ggaccaaat gt t ccagagg 1140
   aaattagggg cccagttgga agcaaggcga gatgactttt gtaagcagaa ttccaaagca    1200
   t cat cagat t gt t gcat ggc t t t act t cag gat at at t t g gccct t t aga agaagat gt c 1260
   aagcagggaa cattttctaa accaggaggt taccgtctct ttactcagaa gctgcaggag    1320
   ctgaagaata agtactacca ggtgccaagg aaggggatac aggccaaaga ggtgctgaaa    1380
   aaat at t t gg agt ccaagga ggat gt ggct gat gcact t c t acagact ga t cagt cact c 1440
   tcagaaaagg aaaaagcgat tgaagtggaa cgtataaagg ctgaatctgc agaagctgca    1500
   aagaaaatgt tggaggaaat acaaaagaag aatgaggaga tgatggaaca gaaagagaag    1560
   agttatcagg aacatgtgaa acaattgact gagaagatgg agagggacag ggcccagtta    1620
   atggcagagc aagagaagac cctcgctctt aaacttcagg aacaggaacg ccttctcaag    1680
   gagggattcg agaatgagag caagagactt caaaaagaca tatgggatat ccagatgaga    1740
   agcaaat cat t ggagccaat at gt aacat a ct t t aa 1776
<210> 10
   <211> 591
   <212> PRT
   <213> Homo sapiens
<400> 10
<210> 11
   <211> 2993
   <212> DNA
   <213> Homo sapiens
<400> 11
<210> 12
   <211> 1923
   <212> DNA
   <213> Homo sapi ens
<400> 12
<210> 13
   <211> 640
   <212> PRT
   <213> Horm sapiens
<400> 13
   Met Gly Glu Arg Thr Leu His Ala Ala Val Pro Thr Pro Gly Tyr Pro
   1 5 10 15
   Glu Ser Glu Ser Ile Met Met Ala Pro Ile Cys Leu Val Glu Asn Gln
   20 25 30
   Glu Glu Gln Leu Thr Val Asn Ser Lys Ala Leu Glu Ile Leu Asp Lys
   35 40 45
   Ile Ser Gln Pro Val Val Val Val Ala Ile Val Gly Leu Tyr Arg Thr
   50 55 60
   Gly Lys Ser Tyr Leu Met Asn Arg Leu Ala Gly Lys Arg Asn Gly Phe
   65 70 75 80
   Pro Leu Gly Ser Thr Val Gln Ser Glu Thr Lys Gly Ile Trp Met Trp
   85 90 95
   Cys Val Pro His Leu Ser Lys Pro Asn His Thr Leu Val Leu Leu Asp
   100 105 110
   Thr Glu Gly Leu Gly Asp Val Glu Lys Ser Asn Pro Lys Asn Asp Ser
   115 120 125
   Trp Ile Phe Ala Leu Ala Val Leu Leu Ser Ser Ser Phe Val Tyr Asn
   130 135 140
   Ser Val Ser Thr Ile Asn His Gln Ala Leu Glu Gln Leu His Tyr Val
   145 150 155 160
   Thr Glu Leu Ala Glu Leu Ile Arg Ala Lys Ser Cys Pro Arg Pro Asp
   165 170 175
   Glu Ala Glu Asp Ser Ser Glu Phe Ala Ser Phe Phe Pro Asp Phe Ile
   180 185 190
   Trp Thr Val Arg Asp Phe Thr Leu Glu Leu Lys Leu Asp Gly Asn Pro
   195 200 205
   Ile Thr Glu Asp Glu Tyr Leu Glu Asn Ala Leu Lys Leu Ile Pro Gly
   210 215 220
   Lys Asn Pro Lys Ile Gln Asn Ser Asn Met Pro Arg Glu Cys Ile Arg
   225 230 235 240
   His Phe Phe Arg Lys Arg Lys Cys Phe Val Phe Asp Arg Pro Thr Asn
   245 250 255
   Asp Lys Gln Tyr Leu Asn His Met Asp Glu Val Pro Glu Glu Asn Leu
   260 265 270
   Glu Arg His Phe Leu Met Gln Ser Asp Asn Phe Cys Ser Tyr Ile Phe
   275 280 285
   Thr His Ala Lys Thr Lys Thr Leu Arg Glu Gly Ile Ile Val Thr Gly
   290 295 300
   Lys Arg Leu Gly Thr Leu Val Val Thr Tyr Val Asp Ala Ile Asn Ser
   305 310 315 320
   Gly Ala Val Pro Cys Leu Glu Asn Ala Val Thr Ala Leu Ala Gln Leu
   325 330 335
   Glu Asn Pro Ala Ala Val Gln Arg Ala Ala Asp His Tyr Ser Gln Gln
   340 345 350
   Met Ala Gln Gln Leu Arg Leu Pro Thr Asp Thr Leu Gln Glu Leu Leu
   355 360 365
   Asp Val His Ala Ala Cys Glu Arg Glu Ala Ile Ala Val Phe Met Glu
   370 375 380
   His Ser Phe Lys Asp Glu Asn His Glu Phe Gln Lys Lys Leu Val Asp
   385 390 395 400
   Thr Ile Glu Lys Lys Lys Gly Asp Phe Val Leu Gln Asn Glu Glu Ala
   405 410 415
   Ser Ala Lys Tyr Cys Gln Ala Glu Leu Lys Arg Leu Ser Glu His Leu
   420 425 430
   Thr Glu Ser Ile Leu Arg Gly Ile Phe Ser Val Pro Gly Gly His Asn
   435 440 445
   Leu Tyr Leu Glu Glu Lys Lys Gln Val Glu Trp Asp Tyr Lys Leu Val
   450 455 460
   Pro Arg Lys Gly Val Lys Ala Asn Glu Val Leu Gln Asn Phe Leu Gln
   465 470 475 480
   Ser Gln Val Val Val Glu Glu Ser Ile Leu Gln Ser Asp Lys Ala Leu
   485 490 495
   Thr Ala Gly Glu Lys Ala Ile Ala Ala Glu Arg Ala Met Lys Glu Ala
   500 505 510
   Ala Glu Lys Glu Gln Glu Leu Leu Arg Glu Lys Gln Lys Glu Gln Gln
   515 520 525
   Gln Met Met Glu Ala Gln Glu Arg Ser Phe Gln Glu Asn Ile Ala Gln
   530 535 540
   Leu Lys Lys Lys Met Glu Arg Glu Arg Glu Asn Leu Leu Arg Glu His
   545 550 555 560
   Glu Arg Leu Leu Lys His Lys Leu Lys Val Gln Glu Glu Met Leu Lys
   565 570 575
   Glu Glu Phe Gln Lys Lys Ser Glu Gln Leu Asn Lys Glu Ile Asn Gln
   580 585 590
   Leu Lys Glu Lys Ile Glu Ser Thr Lys Asn Glu Gln Leu Arg Leu Leu
   595 600 605
   Lys Ile Leu Asp Met Ala Ser Asn Ile Met Ile Val Thr Leu Pro Gly
   610 615 620
   Ala Ser Lys Leu Leu Gly Val Gly Thr Lys Tyr Leu Gly Ser Arg Ile
   625 630 635 640
<210> 14
   <211> 2431
   <212> DNA
   <213> Homo sapiens
<400> 14
   ctccaggctg tggaaccttt gttctttcac tctttgcaat aaatcttgct gctgctcact      60
   ctttgggtcc acactgcctt tatgagctgt aacactcact gggaatgtct gcagcttcac     120
   tcctgaagcc agcgagacca cgaacccacc aggaggaaca aacaactcca gacgcgcagc     180
   cttaagagct gtaacactca ccgcgaaggt ctgcagcttc actcctgagc cagccagacc     240
   acgaacccac cagaaggaag aaactccaaa cacatccgaa catcagaagg agcaaactcc     300
   tgacacgcca cctttaagaa ccgtgacact caacgctagg gtccgcggct tcattcttga     360
   agtcagtgag accaagaacc caccaattcc ggacacgcta attgttgtag atcatcactt     420
   caaggtgccc atatctttct agtggaaaaa ttattctggc ctccgctgca tacaaatcag     480
   gcaaccagaa ttctacatat ataaggcaaa gtaacatcct agacatggct ttagagatcc     540
   acatgtcaga ccccatgtgc ctcatcgaga actttaatga gcagctgaag gttaatcagg     600
   aagctttgga gatcctgtct gccattacgc aacctgtagt tgtggtagcg attgtgggcc     660
   tctatcgcac tggcaaatcc tacctgatga acaagctggc tgggaagaac aagggcttct     720
   ctgttgcatc tacggtgcag tctcacacca agggaatttg gatatggtgt gtgcctcatc     780
   ccaactggcc aaatcacaca ttagttctgc ttgacaccga gggcctggga gatgtagaga     840
   aggctgacaa caagaatgat atccagatct ttgcactggc actcttactg agcagcacct     900
   ttgtgtacaa tactgtgaac aaaattgatc agggtgctat cgacctactg cacaatgtga     960
   cagaactgac agatctgctc aaggcaagaa actcacccga ccttgacagg gttgaagatc    1020
   ctgctgactc tgcgagcttc ttcccagact tagtgtggac tctgagagat ttctgcttag    1080
   gcctggaaat agatgggcaa cttgtcacac cagatgaata cctggagaat tccctaaggc    1140
   caaagcaagg tagtgatcaa agagttcaaa atttcaattt gccccgtctg tgtatacaga    1200
   agttctttcc aaaaaagaaa tgctttatct ttgacttacc tgctcaccaa aaaaagcttg    1260
   cccaacttga aacactgcct gatgatgagc tagagcctga atttgtgcaa caagtgacag    1320
   aattctgttc ctacatcttt agccattcta tgaccaagac tcttccaggt ggcatcatgg    1380
   tcaatggatc tcgtctaaag aacctggtgc tgacctatgt caatgccatc agcagtgggg    1440
   atctgccttg catagagaat gcagtcctgg ccttggctca gagagagaac tcagctgcag    1500
   tgcaaaaggc cattgcccac tatgaccagc aaatgggcca gaaagtgcag ctgcccatgg    1560
   aaaccctcca ggagctgctg gacctgcaca ggaccagtga gagggaggcc attgaagtct    1620
   tcatgaaaaa ctctttcaag gatgtagacc aaagtttcca gaaagaattg gagactctac    1680
   tagatgcaaa acagaatgac atttgtaaac ggaacctgga agcatcctcg gattattgct 1740
   cggctttact taaggatatt tttggtcctc tagaagaagc agtgaagcag ggaatttatt 1800
   ctaagccagg aggccataat ctcttcattc agaaaacaga agaactgaag gcaaagtact 1860
   atcgggagcc tcggaaagga atacaggctg aagaagttct gcagaaatat ttaaagtcca 1920
   aggagtctgt gagtcatgca atattacaga ctgaccaggc tctcacagag acggaaaaaa 1980
   agaagaaaga ggcacaagtg aaagcagaag ctgaaaaggc tgaagcgcaa aggttggcgg 2040
   cgattcaaag gcagaacgag caaatgatgc aggagaggga gagactccat caggaacaag 2100
   tgagacaaat ggagatagcc aaacaaaatt ggctggcaga gcaacagaaa atgcaggaac 2160
   aacagatgca ggaacaggct gcacagctca gcacaacatt ccaagctcaa aatagaagcc 2220
   ttctcagtga gctccagcac gcccagagga ctgttaataa cgatgatcca tgtgttttac 2280
   tctaaagtgc taaatatggg agtttccttt ttttactctt tgtcactgat gacacaacag 2340
   aaaagaaact gtagaccttg ggacaatcaa catttaaata aactttataa ttattttttc 2400
   aaactttaaa aaaaaaaaaa aaaaaaaaaa a 2431
<210> 15
   <211> 586
   <212> PRT
   <213> Homo sapiens
<400> 15
   Met Ala Leu Glu Ile His Met Ser Asp Pro Met Cys Leu Ile Glu Asn
   1 5 10 15
   Phe Asn Glu Gln Leu Lys Val Asn Gln Glu Ala Leu Glu Ile Leu Ser
   20 25 30
   Ala Ile Thr Gln Pro Val Val Val Val Ala Ile Val Gly Leu Tyr Arg
   35 40 45
   Thr Gly Lys Ser Tyr Leu Met Asn Lys Leu Ala Gly Lys Asn Lys Gly
   50 55 60
   Phe Ser Val Ala Ser Thr Val Gln Ser His Thr Lys Gly Ile Trp Ile
   65 70 75 80
   Trp Cys Val Pro His Pro Asn Trp Pro Asn His Thr Leu Val Leu Leu
   85 90 95
   Asp Thr Glu Gly Leu Gly Asp Val Glu Lys Ala Asp Asn Lys Asn Asp
   100 105 110
   Ile Gln Ile Phe Ala Leu Ala Leu Leu Leu Ser Ser Thr Phe Val Tyr
   115 120 125
   Asn Thr Val Asn Lys Ile Asp Gln Gly Ala Ile Asp Leu Leu His Asn
   130 135 140
   Val Thr Glu Leu Thr Asp Leu Leu Lys Ala Arg Asn Ser Pro Asp Leu
   145 150 155 160
   Asp Arg Val Glu Asp Pro Ala Asp Ser Ala Ser Phe Phe Pro Asp Leu
   165 170 175
   Val Trp Thr Leu Arg Asp Phe Cys Leu Gly Leu Glu Ile Asp Gly Gln
   180 185 190
   Leu Val Thr Pro Asp Glu Tyr Leu Glu Asn Ser Leu Arg Pro Lys Gln
   195 200 205
   Gly Ser Asp Gln Arg Val Gln Asn Phe Asn Leu Pro Arg Leu Cys Ile
   210 215 220
   Gln Lys Phe Phe Pro Lys Lys Lys Cys Phe Ile Phe Asp Leu Pro Ala
   225 230 235 240
   His Gln Lys Lys Leu Ala Gln Leu Glu Thr Leu Pro Asp Asp Glu Leu
   245 250 255
   Glu Pro Glu Phe Val Gln Gln Val Thr Glu Phe Cys Ser Tyr Ile Phe
   260 265 270
   Ser His Ser Met Thr Lys Thr Leu Pro Gly Gly Ile Met Val Asn Gly
   275 280 285
   Ser Arg Leu Lys Asn Leu Val Leu Thr Tyr Val Asn Ala Ile Ser Ser
   290 295 300
   Gly Asp Leu Pro Cys Ile Glu Asn Ala Val Leu Ala Leu Ala Gln Arg
   305 310 315 320
   Glu Asn Ser Ala Ala Val Gln Lys Ala Ile Ala His Tyr Asp Gln Gln
   325 330 335
   Met Gly Gln Lys Val Gln Leu Pro Met Glu Thr Leu Gln Glu Leu Leu
   340 345 350
   Asp Leu His Arg Thr Ser Glu Arg Glu Ala Ile Glu Val Phe Met Lys
   355 360 365
   Asn Ser Phe Lys Asp Val Asp Gln Ser Phe Gln Lys Glu Leu Glu Thr
   370 375 380
   Leu Leu Asp Ala Lys Gln Asn Asp Ile Cys Lys Arg Asn Leu Glu Ala
   385 390 395 400
   Ser Ser Asp Tyr Cys Ser Ala Leu Leu Lys Asp Ile Phe Gly Pro Leu
   405 410 415
   Glu Glu Ala Val Lys Gln Gly Ile Tyr Ser Lys Pro Gly Gly His Asn
               420                 425                 430
   Leu Phe Ile Gln Lys Thr Glu Glu Leu Lys Ala Lys Tyr Tyr Arg Glu
           435                 440                 445
   Pro Arg Lys Gly Ile Gln Ala Glu Glu Val Leu Gln Lys Tyr Leu Lys
       450                 455                 460
   Ser Lys Glu Ser Val Ser His Ala Ile Leu Gln Thr Asp Gln Ala Leu
   465                 470                 475                 480
   Thr Glu Thr Glu Lys Lys Lys Lys Glu Ala Gln Val Lys Ala Glu Ala
                   485                 490                 495
   Glu Lys Ala Glu Ala Gln Arg Leu Ala Ala Ile Gln Arg Gln Asn Glu
               500                 505                 510
   Gln Met Met Gln Glu Arg Glu Arg Leu His Gln Glu Gln Val Arg Gln
           515                 520                 525
   Met Glu Ile Ala Lys Gln Asn Trp Leu Ala Glu Gln Gln Lys Met Gln
       530                 535                 540
   Glu Gln Gln Met Gln Glu Gln Ala Ala Gln Leu Ser Thr Thr Phe Gln
   545                 550                 555                 560
   Ala Gln Asn Arg Ser Leu Leu Ser Glu Leu Gln His Ala Gln Arg Thr
                   565                 570                 575
   Val Asn Asn Asp Asp Pro Cys Val Leu Leu
               580                 585